# EUROPÄISCHE PATENTSCHRIFT

(11) **EP 2 356 088 B1**
(45) Veröffentlichungstag und Bekanntmachung des Hinweises auf die Patenterteilung: **26.09.2018**
(21) Anmeldenummer: 09745047.2
(22) Anmeldetag: 03.11.2009
(51) Int. Cl.: C07C 45/52, C07C 45/78, C07C 45/79, C07C 45/82, C07C 47/22, B01J 19/00, C07C 57/055, D01F 6/16, D06M 16/00, C08F 20/06

(54) **VERFAHREN ZUR HERSTELLUNG VON ACROLEIN UMFASSEND DIE AUFARBEITUNG EINER ROHGLYCERIN-PHASE**
METHOD FOR PRODUCING ACROLEIN COMPRISING THE REGENERATION OF A RAW GLYCERIN PHASE
PROCÉDÉ DE PRODUCTION D'ACROLÉINE COMPORTANT LE TRAITEMENT D'UNE PHASE GLYCÉRINE BRUTE

(30) Priorität: 09.12.2008 DE 102008060888
(43) Veröffentlichungstag der Anmeldung: 17.08.2011
(73) Patentinhaber: Evonik Degussa GmbH, 45128 Essen (DE)
(72) Erfinder: STOCHNIOL, Guido, 45721 Haltern am See (DE); BAUMGARTEN, Götz, 45721 Haltern am See (DE); KUPPINGER, Franz-Felix, 45768 Marl (DE)
(86) Internationale Anmeldenummer: PCT/EP2009/064500
(87) Internationale Veröffentlichungsnummer: WO 2010/066513

(56) Entgegenhaltungen:
- EP-A1- 0 358 255
- EP-A1- 0 959 062
- WO-A2-2006/087083
- WO-A2-2008/023040
- DE-A1- 10 138 150
- DE-A1- 19 636 767
- DE-A1-102007 004 350
- DE-A1-102007 019 379
- DE-C1- 4 238 493
- US-A- 2 802 861
- US-A- 3 919 306
- US-A- 5 250 182

## Beschreibung

Gegenstand der Erfindung sind allgemein ein Verfahren zur Herstellung von Acrolein oder Acrylsäure.

Acrylsäure ist ein wichtiger Grundstoff in der Kunststoffindustrie, da es zu Polyacrylaten und Copolymerisaten weiterverarbeitet werden kann. Acrolein dient als Ausgangsstoff in der industriellen Synthese von Acrylsäure. Ein wichtiges industrielles Verfahren zur Herstellung von Acrolein ist die partielle Oxidation von Glycerin. Dieses fällt in großen Mengen bei der Spaltung oder Umesterung von Glyceriden an.

Bei solchen Glycerid-Spaltprozessen oder Glycerid-Umesterungsprozessen fällt ein etwa 70 bis 90, oftmals auch 75 bis 85 meist jedoch etwa 80-prozentiges Roh-glycerin an, das je nach dem verwendeten Herstellungsprozess weitere Komponenten enthält. Der größte Nebenbestandteil ist mit oftmals etwa ab 1% bis zu etwa 20% Wasser. Darüber hinaus sind in der Regel Natrium- und Kaliumssalze verschiedener Mineralsäuren enthalten, beispielsweise Salze der Salz-, Schwefel- oder Phosphorsäure. In einigen Fällen liegen auch Salze organischer Säuren, beispielsweise der Zitronensäure, vor. Zusätzlich finden sich freie Fettsäuren und Seifen im Rohglycerin. Die unerwünschten organischen Bestandteile werden als MONG (für "*matter organic non-glycerol*") bezeichnet.

Bei der Verwendung des so erzeugten Rohglycerins in nachfolgenden Syntheseprozessen im industriellen Maßstab bewirken die Salze, insbesondere Chloride, Korrosionsprobleme. Sie müssen daher aus dem Rohglycerin entfernt werden. Die organischen Bestandteile des Rohglycerins können bei der nachfolgenden Synthese des Acroleins zu unerwünschten Nebenprodukten und zu Problemen bei der Aufreinigung führen. Außerdem können Sie, wie auch die Salze, in unerwünschter Form auf Katalysatoren einwirken und zu Verkokungen führen.

Ein Verfahren zur Herstellung von Acrolein aus Glycerin und zur weiteren Verarbeitung zu Acrylsäure ist aus der WO-A-2006/092272 bekannt. Dass Glycerin wird dabei durch die Spaltung von Triglyceriden gewonnen und direkt zu Acrolein weiterverarbeitet. Die Abtrennung des Acroleins von der Reaktionsmischung erfolgt durch eine Destillation. Bei diesem Prozess werden auch schwer siedende Bestandteile entfernt. Ein verbleibendes Gemisch, das hohe Anteile an Wasser und Glycerin enthält, wird in das ursprüngliche Reaktionsgemisch zur Synthese von Acrolein zurückgeführt.

Ein weiteres Verfahren wird in der DE-A-10 2005 028 624 beschrieben. Dabei wird Acrolein aus einer wässrigen Glycerinphase hergestellt. Aus dem erhaltenen Reaktionsprodukt wird das Acrolein abgereichert. Die abgereicherte Reaktionsphase wird in einem Kreislauf in den Reaktionsbereich zurückgeführt, so dass das nicht umgesetzte Glycerin zu Acrolein umgesetzt werden kann.

Die Verfahren der WO-A-2006/092272 und DE-A-10 2005 028 624 weisen den Vorteil auf, dass wegen des Kreislaufs, durch den das nicht umgesetzte Glycerin wieder der Synthese zugeführt wird, insgesamt ein hoher Anteil des eingesetzten Glycerins umgesetzt wird.

Nachteilig ist jedoch, dass bei diesen Verfahren ein Teil der unerwünschten Nebenprodukte, insbesondere die Salze und die Chlorid-Ionen, den großindustriellen Langzeiteinsatz stören und zu vermehrten Stillstandszeiten führen. Daher wird in der WO-A-2006/092272 vorgeschlagen, das Wasser und Glycerin aufweisende Gemisch aus dem Schwersiederabscheider mit einer Membran zu reinigen. Dadurch wird der beschriebene Nachteil jedoch nur zum Teil behoben. Viele Bestandteile, insbesondere die Salze und die Chlorid-Ionen, lassen sich mit einer solchen Membran nicht oder nicht vollständig abtrennen. Es kann daher das Problem auftreten, dass sich bei längerer Reaktionsführung zunehmend unerwünschte Bestandteile in dem Kreislauf ansammeln, die die Reaktion beeinträchtigen oder Teile der Vorrichtung durch Korrosion schädigen.

WO 2008/023040A2 offenbart ein Verfahren zur Herstellung von Acrolein durch dehydratisieren von Glycerin und die Gasphasenoxidation des Dehydratisierungsproduktes unter Erhalt von Acrylsäure. Zwischen dem Dehydratisierungsreaktor

DE 10 2007 004350 A1 offenbart ein Verfahren zur Regenerierung eines Katalysators, der zur Herstellung von Acrolein aus Glycerin verwendet wird.

DE 42 38 493 C1 offenbart ein Verfahren zur Herstellung von Acrolein und dessen Verwendung

US 2,802,861 A offenbart eine Vorrichtung umfassend einen Reaktor und ein Mittel zur Zuführung einer Lösung zu dem Reaktor, eine Destillationseinheit mit einem Abtrennungsmittel, eine Filtereinheit als Reinigungsmittel und eine Rückführleitung.

DE 196 36 767 offenbart eine Vorrichtung umfassend einen Reaktor R1 und ein Mittel zur Zuführung einer Lösung zu dem Reaktor, mindestens zwei Destillationskolonnen mit einem Abtrennungsmittel, eine Rückführleitung und einen zweiten Reaktor R2.

US 3,919,306 A offenbart eine Vorrichtung umfassend einen Oxidationsreaktor, ein Mittel zur Zuführung einer Lösung zu dem Reaktor, eine Flashdestillationskolonne mit einem Abtrennungsmittel, eine "rotary filtering zone" als Abtrennungsmittel und eine Rückführleitung.

WO 2006/087083 A2 offenbart die Vorbehandlung von Rohglycerin mittels Ionenaustausch, da die Anwesenheit von Natriumsalzen besonders nachteilig für die katalytische Dehydratisierungsreaktion sein soll.

EP 0 358 255 A1 offenbart Reinigungsverfahren zur Reinigung von rohem Glycerin, das aus der Methanolyse stammt. Diese Verfahren umfassen Destillation, Behandlung Ionenaustauscherharzen und Mikrofiltration oder Ultrafiltration.

Der vorliegenden Erfindung liegt die Aufgabe zu Grunde, mindestens einen der sich aus dem Stand der Technik ergebenden Nachteile zumindest teilweise zu überwinden.

Des Weiteren sollen verbesserte Verfahren zur Herstellung von Acrylsäure und Polymerisationsprodukten aus Acrylsäure, wie wasserabsorbierenden Polymergebilden, bereitgestellt werden, die sich besonders für den großtechnischen Einsatz eignen. Es sollen Verfahren bereitgestellt werden, die auf der einen Seite möglichst einfach und effizient durchführbar sind und bei denen auf der anderen Seite möglichst wenig unerwünschte Nebenprodukte anfallen oder sich bei der Durchführung des Verfahrens ansammeln. Das erfindungsgemäße Verfahren soll es ermöglichen, in einer Vorrichtung die Reaktionsbestandteile möglichst effizient zu verwerten. Insbesondere sollen sich in einem erfindungsgemäßen Verfahren möglichst wenig Salze und Chlorid-Ionen ansammeln, so dass Korrosionsschäden und Beeinträchtigungen der Oxidationsreaktion nicht auftreten oder zumindest verringert werden und das bei einem einfachen und ohne große Unterberechungen erfolgenden Betrieb.

Eine weitere der Erfindung zugrunde liegende Aufgabe ist es, ein integriertes Verfahren zur Herstellung von Acrolein und Folgeprodukten bereitzustellen, bei dem dieser Herstellungsprozess möglichst störungsfrei in einem Kreislauf durchgeführt werden kann.

Eine weitere der Erfindung zugrunde liegende Aufgabe ist es, bereits auf der Stufe der eingespeisten Glycerinlösung der Ansammlung von Nebenprodukten, wie Salzen und organischen Bestandteilen, entgegenzuwirken, so dass das Verfahren auch den Einsatz von Rohglycerin, welches beispielsweise als Nebenprodukt bei der Biodieselherstellung anfällt, ermöglicht.

Eine weitere Aufgabe der Erfindung ist es, den Prozess in optimaler Weise mit vor- und nachgeschalteten Prozessen zu verbinden, so dass die Durchführung des Verfahrens in einem einzigen integrierten Prozess und/oder mit einer einzigen Vorrichtung möglich ist. Dies betrifft insbesondere Verfahren zur Herstellung von Acrylsäure und Polymerisationsprodukten aus Acrylsäure, wie wasserabsorbierenden Polymergebilden.

Eine weitere Aufgabe der Erfindung ist es, ein Verfahren zur Herstellung von Acrolein und Folgeprodukten bereitzustellen, bei dem glycerinhaltige Ausgangslösungen und Zwischenprodukte, insbesondere das bei der Fettspaltung oder der Biodieselherstellung anfallende Rohglycerin, auf möglichst wenig kosten- und energieintensive und gleichwohl auf eine für die großindustrielle Anwendung geeigneten Art gereinigt und zur Herstellung von Acrolein und Folgeprodukten davon eingesetzt werden können. Dieses gilt insbesondere im Zusammenhang mit einer möglichst kontinuierlichen Verfahrenführung.

Auch lag der vorliegenden Erfindung die Aufgabe zugrunde, ein Verfahren zur Aufreinigung einer Glycerinphase, vorzugsweise von bei Glycerin-Spaltprozessen oder Glycerin-Umesterungsprozessen anfallendem Roh-Glycerin, anzugeben, mit dem Glycerinphasen besonders effektiv aufgereinigt werden können.

Einen Beitrag zur Lösung mindestens einer dieser Aufgaben leistet der Gegenstand der kategoriebildenden Ansprüche, wobei Neben- und Unteransprüche bevorzugte Ausführungsformen der vorliegenden Erfindung darstellen.

Einen Beitrag leistet insbesondere ein erfindungsgemäßes Verfahren zur Herstellung von Acrolein, beinhaltend die folgenden Schritte:
(a) Dehydratisieren einer wässrigen Glycerinphase G1 in einem Acroleinreaktionsbereich unter Erhalt einer wässrigen Acroleinreaktionsphase;
(b) mindestens teilweises Auftrennen der wässrigen Acroleinreaktionsphase in eine Acrolein-reiche Acroleinphase und eine im Vergleich zu der Acroleinphase Acrolein-arme Restphase R1, wobei die Restphase R1 Glycerin, Wasser und von Glycerin und Wasser verschiedene Reststoffe beinhaltet;
(c) Rückführen mindestens eines Teils der Restphase R1 in Schritt (a);
wobei
i) aus einer Glycerinphase G2, beinhaltend Wasser, Glycerin und von Glycerin und Wasser verschiedene Reststoffe, mindestens einer dieser Reststoffe abgetrennt und die so erhaltene, aufgereinigte Glycerinphase G2 unmittelbar dem Acroleinreaktionsbereich zugeführt wird, oder
ii) aus einer Mischphase M1, erhalten durch das Vermischen einer Glycerin-phase G2, beinhaltend Wasser, Glycerin und von Glycerin und Wasser verschiedene Reststoffe, mit der Acrolein-armen Restphase R1, mindestens einer der von Glycerin und Wasser verschiedenen Reststoffe abgetrennt und die so erhaltene, aufgereinigte Mischphase M1 dem Acroleinreaktionsbereich zugeführt wird,
wobei neben der aufgereinigten Glycerinphase G2 oder der aufgereinigten Mischphase M1 eine weitere Glycerinphase G3 dem Acroleinreaktionsbereich zugeführt wird, wobei die Glycerinphase G3 einen höheren Glyceringehalt als die Glycerin-phase G2, und wobei die Glycerinphase G3 einen geringeren Salzgehalt als die Glycerinphase G2 und wobei die Glycerinphase G3 einen geringeren Gehalt an von Glycerin verschiedenen organischen Verbindungen als die Glycerinphase G2 aufweist, und wobei die Glycerinphase G3 als Destillat bei der Destillation von bei der Spaltung oder der Umesterung von Triglyceriden erhaltenem Rohglycerin erhalten wird und mindestens ein Teil des bei der Destillation des Rohglycerins anfallenden Sumpfproduktes einen Salzabscheider durchläuft und anschließend der Destillation des Rohglycerins wieder zugeführt wird.

Als Reststoffe, welche in der Acrolein-armen Restphase R1 enthalten sind, kommen grundsätzlich alle bei der Dehydratisierung von Glycerin zu Acrolein anfallenden Nebenprodukte sowie diejenigen Nebenprodukte, welche in der im Verfahrensschritt (a) eingesetzten wässrigen Glycerinphase G1 enthalten sind bzw. deren bei der Dehydratisierung gegebenenfalls gebildeten Folgeprodukte in Betracht. Der Anteil der Reststoffe liegt in der Regel in einem Bereich von 0,001 bis 20 Gew.-%, vorzugsweise in einem Bereich von 0,001 bis 10 Gew.-% und besonders bevorzugt in einem Bereich von 0,001 bis 5 Gew.-%, jeweils bezogen auf die Acrolein-arme Restphase R1. Besondere Bedeutung haben Feststoffe, wie Teer- bzw. Verkokungsprodukte, Oligomere des Acroleins sowie Oligomer des Glycerins oder auch die Teer- bzw. Verkokungsprodukte dieser Oligomere.

Einen weiteren erfindungsgemäßen Beitrag leistet ein Verfahren zur Herstellung von Acrylsäure, beinhaltend die Schritte:
(A1) Bereitstellen einer Acrolein-reichen Acroleinphase nach dem vorstehend beschrieben erfindungsgemäßen Verfahren zur Herstellung von Acrolein;
(A2) Oxidation, vorzugsweise Gasphasenoxidation an einem Gasphasenkatalysator, der Acrolein-reichen Acroleinphase aus Schritt (A1) unter Erhalt einer Acrylsäurephase; und
(A3) gegebenenfalls Aufarbeiten der Acrylsäurephase unter Erhalt von Acrylsäure.

Zudem leistet einen Beitrag ein Verfahren zur Herstellung eines Polymers, beinhaltend die Schritte:
(P1) Bereitstellen einer Acrylsäurephase oder einer Acrylsäure nach dem beschriebenen erfindungsgemäßen Verfahren zur Herstellung von Acrylsäure in einer Polymeristionsphase;
(P2) Polymerisieren der Polymerisationsphase unter Erhalt eines Polymers.

Allgemein sind Verfahren zur Herstellung von Acrolein aus dem Stand der Technik bekannt. Diesbezüglich wird auf die WO-A-2006/092272 und die DE-A-10 2005 028 624 verwiesen. Auf den Gegenstand beider Anmeldungen, insbesondere die offenbarten Verfahren zur Synthese von Acrolein, Acrylsäure und wasserabsorbierenden Polymergebilden und die Vorrichtungen wird hier ausdrücklich Bezug genommen. Die erfindungsgemäße Reinigung gemäß den Alternativen i) und ii) kann bei diesen Verfahren eingesetzt werden.

Im Schritt (a) des erfindungsgemäßen Verfahrens zur Herstellung von Acrolein wird eine wässrige Glycerinphase G1 in einem Acroleinreaktionsbereich unter Erhalt einer wässrigen Acroleinreaktionsphase dehydratisiert. Mit dem Begriff "*Acroleinreaktionsbereich*" wird dabei der Teil der Vorrichtung bezeichnet, in dem die Dehydratisierung des Glycerins zu Acrolein durchgeführt wird.

Die Dehydratisierung des Glycerins kann in einer bevorzugten Ausführungsform der Erfindung in einer flüssigen Phase stattfinden. In einer weiteren bevorzugten Ausführungsform der Erfindung erfolgt die Dehydratisierung in der Gasphase. Die Dehydratisierung kann auch in einem kombinierten Verfahren durchgeführt werden, in dem die Flüssig- und Gasphasendehydratisierung kombiniert werden. In diesem Zusammenhang wird ausdrücklich Bezug genommen auf die entsprechenden Verfahren, die in der WO-A-2006/092272 offenbart werden.

Es ist erfindungsgemäß bevorzugt, dass der Druck im Acroleinreaktionsbereich mindestens 50, vorzugsweise mindestens 80 und besonders bevorzugt mindestens 120 sowie darüber hinaus bevorzugt mindestens 140 bar beträgt. Der Acroleinreaktionsbereich ist somit als Druckbereich ausgestaltet, der an seinem Beginn durch einen Druckerzeuger, wie einer Pumpe, und an seinem Ende durch einen Druckregler, wie ein Druckventil und darüber hinaus bevorzugt ein Druckregelventil, abgegrenzt ist. Die Dehydratisierungsreaktion im Verfahrensschritt (a) erfolgt mindestens in einem Teil des Acroleinreaktionsbereichs. Üblicherweise ist der Acroleinreaktionsbereich mindestens teilweise rohrartig ausgebildet und bis zu einer maximalen Druckbelastung von 500 bar und einer maximalen Temperaturbelastung von 600°C ausgelegt, die zur Durchführung des erfindungsgemäßen Verfahrens ausreichen.

Zudem ist es erfindungsgemäß bevorzugt, dass die Temperatur im Acroleinreaktionsbereich mindestens 80°C, vorzugsweise mindestens 180°C, besonders bevorzugt mindestens 230°C und darüber hinaus bevorzugt mindestens 280°C sowie ferner bevorzugt mindestens 320°C beträgt. Die Temperaturen können zum einen über die Druckverhältnisse in dem Acroleinreaktionsbereich als auch über eine entsprechende Beheizung des Acroleinreaktionsbereiches erreicht werden. Im Allgemeinen werden die Druck- und/oder Temperaturbedingungen im Acroleinreaktionsbereich so ausgewählt, dass sich die Acroleinreaktionsphase und insbesondere das darin enthaltene Wasser zumindest nahe des oder mindestens teilweise im überkritischen Bereich befinden.

Zudem ist es in dem erfindungsgemäßen Verfahren bevorzugt, dass der Acroleinreaktionsbereich neben dem Reaktionsgemisch einen Dehydratisierungskatalysator aufweist. Dieser liegt vorzugsweise in einer Menge im Bereich von 0,001 : 1000 bis 10 : 1000, bevorzugt von 0,01 : 1000 bis 5 : 1000 und besonders bevorzugt von 0,04 : 1000 bis 1 : 1000, jeweils bezogen auf die in der Acroleinreaktionsphase eingesetzte Glycerinmenge, vor.

In einer bevorzugten Ausführungsform des erfindungsgemäßen Verfahrens kann der Dehydratisierungskatalysator entweder als Säure oder als Base oder als eine Kombination daraus vorliegen. Wenn der Dehydratisierungskatalysator als Säure vorliegt, handelt es sich um Verbindungen, die neben Wasser, das nahe dem oder in dem überkritischen Bereich ebenfalls als starke Säure agiert, saure Eigenschaften besitzen. Wenn der Dehydratisierungskatalysator eine Säure ist, kommen sowohl anorganische als auch organische Säuren in Betracht. Als anorganische Säuren kommen insbesondere Säuren des Phosphors wie H₃PO₄, des Schwefels wie H₂SO₄, des Bors wie B(OH)₃ oder eine Mischung daraus in Betracht. In einer weiteren Ausgestaltung des Dehydratisierungskatalysators liegt dieser als Supersäure, die definitionsgemäß einen kleinen pKs-Wert von < -1 aufweist, vor. Wenn der Dehydratisierungskatalysator als organische Säure vorliegt, sind Alkylsulfonsäuren bevorzugt, wobei Trifluormethansulfonsäure oder Methansulfonsäure oder deren Mischungen besonders bevorzugt sind. Als Basen kommen im Zusammenhang mit dem Dehydratisierungskatalysator insbesondere Aluminium-, Lanthan-, Alkali- oder Erdalkalioxide, -hydroxyde, -phosphate, -pyrophosphate, -hydrogenphosphate oder -carbonate oder eine Mischung aus mindestens zwei davon, die jeweils auch geträgert sein können, in Betracht.

Weiterhin kann der Dehydratisierungskatalysator bei Raumtemperatur sowohl als Feststoff als auch als Flüssigkeit vorliegen. Unter als Feststoff vorliegender Dehydratisierungskatalysator fallen auch an einen Feststoffträger immobilisierte, flüssige Dehydratisierungskatalysatoren. Bevorzugte feste Dehydratisierungskatalysatoren sind insbesondere Siliziumoxid beinhaltende Verbindungen wie Zeolithe. Es kommen außerdem Ti-, Zr- oder Ce-Oxide, sulfatisierte Oxide sowie phosphatisierte Oxide oder Mischungen aus mindestens zwei davon in Betracht. Eine Reihe von Dehydratisierungskatalysatoren werden im Einzelnen in DE-A-42 38 493 beschrieben, so dass in diesem Zusammenhang auf die Offenbarung dieses Dokuments Bezug genommen wird.

Weiterhin ist es in dem erfindungsgemäßen Verfahren bevorzugt, dass die Acroleinreaktionsphase eine von Wasser verschiedene Flüssigkeit aufweist. Für den Fall, dass flüssige Dehydratisierungskatalysatoren eingesetzt werden, sollte sich diese Flüssigkeit ebenso auch von diesen unterscheiden. Diese Flüssigkeiten haben eine Funktion als Löslichkeitsverbesserer. Allgemein kommen als solche Flüssigkeiten bei 20°C mit Wasser mischbare organische Verbindungen in Betracht, die mindestens ein Heteroatom, vorzugsweise zwei Heteroatome, aufweisen und gegenüber den anderen Bestandteilen der Acroleinreaktionsphase inert sind. Solche Flüssigkeiten sind beispielsweise Hydroxypiperidin oder aprotische und polare Flüssigkeiten, wie Sulfolan, Diglyme, Tetraglyme, Dioxan, Trioxan oder γ-Butyrolacton. Weiterhin kommen als Flüssigkeiten Verbindungen oder aber Lösungen von Verbindung in Betracht, die eine chelatisierende Wirkung zeigen. In diesem Zusammenhang kommen beispielsweise EDTA, NTA oder DPTA, wie sie etwa unter den Handelsnamen Versene®, Versenex®, Entarex® oder Detarex ® erhältlich sind, oder auch Kronenether in Betracht.

Weiterhin ist es in dem erfindungsgemäßen Verfahren bevorzugt, dass der Acroleinreaktionsbereich ein Metall oder eine Metallverbindung oder beides beinhaltet. Hierbei handelt es sich vorzugsweise um ein-, zwei- oder mehrwertige Metalle oder Metallverbindungen. Zudem ist es bevorzugt, dass dieses Metall oder diese Metallverbindungen sich von dem oder den Metallen, die zum Bau des Acroleinreaktionsbereichs verwendet werden, unterscheiden. Gleichwohl entspricht es einer erfindungsgemäßen Ausführungsform, dass diese Metalle oder Metallverbindungen an dem für den Bau des Acroleinreaktionsbereichs verwendeten Material unmittelbar oder mittelbar unter Zuhilfenahme eines Haftvermittlers immobilisiert sind. Diese Metalle oder Metallverbindungen können jedoch auch partikulär in dem Acroleinreaktionsbereich vorliegen. Allgemein ist es bevorzugt, dass diese Metalle oder Metallverbindungen nicht durch einen Flüssigkeits- oder Gasstrom aus dem Acroleinreaktionsbereich austragbar sind. Dieses kann neben der Immobilisierung dieser Metalle oder Metallverbindungen im Fall des partikulären Vorliegens durch geeignete, im Acroleinreaktionsbereich vorgesehene Siebe oder Filter erreicht werden. Ferner entspricht es einer Ausgestaltung des erfindungsgemäßen Verfahrens, dass die Metalle bzw. Metallverbindungen so ausgewählt sind, dass die vorstehend erwähnten Flüssigkeiten an diesen Metallen oder Metallverbindungen koordinieren oder gar komplexieren können. Zudem ist es in dem erfindungsgemäßen Verfahren bevorzugt, dass diese Metalle als Metallverbindungen vorliegen, wobei Metallsalze oder mit Liganden komplexierte Metalle besonders bevorzugt sind. Als Liganden kommen insbesondere Kohlenmonoxyd wie Carbonyl, Triphenylphosphin, Cp, Cp* oder AcAc in Betracht. Die Metallsalze werden insbesondere in Form ihrer Sulfate oder Phosphate eingesetzt. Als Metalle sind Zinn, insbesondere als Zinnsulfat, Zink, insbesondere als Zinksulfat, Lithium, insbesondere als Lithiumsulfat, Magnesium, insbesondere als Magnesiumsulfat, Kupfer, insbesondere als Kupfersulfat, Palladium, insbesondere als Palladiumcarbonylkomplex, das meist als Acetat eingesetzt wird, Rhodium, insbesondere als Rhodiumcarbonylkomplex, das meist als Acetat eingesetzt wird, Ruthenium, insbesondere als Rhutheniumcarbonylkomplex, das meist als Acetat eingesetzt wird, Nickel, insbesondere als Nickelcarbonylkomplex, das meist als Acetat eingesetzt wird, Eisen, insbesondere als Eisencarbonylkomplex, Kobalt, insbesondere als Kobaltcarbonylkomplex, Cäsium, insbesondere als Cäsiumacetat sowie Lanthanoide, insbesondere Lanthan oder eine Mischung aus mindestens zwei davon zu nennen. Vorzugweise werden die Metalle als Salze mit komplexierenden Agentien, oftmals auch in Gegenwart von Kohlenmonoxid eingesetzt. Als Metallverbindungen sind zudem Heteropolysäuren zu nennen. Bei diesen Heteropolysäuren sind diejenigen bevorzugt, die entstehen, wenn verschiedenartige Säuremoleküle eines Metalls, wie des Chroms, des Wolframs oder des Molybdäns und eines Nichtmetalls, vorzugsweise des Phosphors, unter Austritt von Wasser zusammentreten. Heteropolysäuren sind beispielsweise Phosphorwolframsäuren, Silikowolframsäuren oder Silikomolybdänsäuren und auch die entsprechenden Vanadiumverbindungen.

Ferner ist es in den erfindungsgemäßen Verfahren bevorzugt, dass die Verweilzeit der Acroleinreaktionsphase in dem Acroleinreaktionsbereich im Bereich von 1 bis 10.000, vorzugsweise im Bereich von 5 bis 1.000 und besonders bevorzugt im Bereich von 10 bis 500 Sekunden liegt.

Zudem hat es sich in den erfindungsgemäßen Verfahren als hilfreich erwiesen, dass die Acroleinreaktionsphase Kohlenmonoxid in einem Bereich von 0.0001 bis 10 Gew.-%, vorzugsweise von 0,001 bis 7 Gew.-% und darüber hinaus bevorzugt von 0.005 bis 5 Gew.-%, jeweils bezogen auf die Acroleinreaktionsphase, aufweist. Diese Maßnahme kann für die Verminderung von Nebenkomponenten vorteilhaft sein.

Zudem ist es in dem erfindungsgemäßen Verfahren bevorzugt, dass die Acroleinreaktionsphase am Ende des Acroleinreaktionsbereichs einen Anteil von < 50, vorzugsweise < 25 und darüber hinaus bevorzugt < 15 Gew.-%, jeweils bezogen auf die Acroleinreaktionsphase, Glycerin beinhaltet. Durch diese Verfahrensführung wird nach Durchführung des Verfahrensschrittes (b) eine Acroleinphase erhalten, die sich über einen wesentlich längeren Zeitraum in die Stufe (A2) des erfindungsgemäßen Verfahrens zur Herstellung von Acrylsäure einspeisen lässt, ohne dass es zu nennenswerten Verschlechterungen der Umsetzung von Acrolein zu Acrylsäure kommt. Weiterhin ist es in den erfindungsgemäßen Verfahren allgemein der Fall, dass die Glycerinkonzentration am Beginn des Acroleinreaktionsbereichs größer ist als am Ende des Acroleinreaktionsbereichs und dabei zum Ende hin vorzugsweise kontinuierlich abnimmt. Auch ist es erfindungsgemäß bevorzugt, dass die Acroleinreaktionsphase am Ende des Acroleinreaktionsbereichs einen Anteil im Bereich von 0,1 bis 50 Gew.-%, bevorzugt 5 bis 45 Gew.% und noch mehr bevorzugt 10 bis 40 Gew.-%, jeweils bezogen auf die Acroleinreaktionsphase, Acrolein beinhaltet.

Weiterhin ist es in den erfindungsgemäßen Verfahren bevorzugt, dass mindestens ein Teil der Acroleinreaktionsphase gasförmig vorliegt. Es ist ferner bevorzugt, dass die Acroleinreaktionsphase in dem Acroleinreaktionsbereich in mindestens zwei Aggregatzuständen vorliegt. Bei diesen Aggregatzuständen handelt es sich vorzugsweise um flüssig und gasförmig. Für den Fall, dass mindestens ein Teil der Acroleinreaktionsphase als Gas vorliegt, ist es bevorzugt, dass die Konzentration an Acrolein in dieser Acroleinreaktionsgasphase höher ist als in dem Teil der Acroleinreaktionsphase, der einen von der Acroleinreaktionsgasphase verschiedenen Aggregatzustand besitzt. Durch die hohe Acroleinkonzentration in der Acroleinreaktionsgasphase ist eine Abreicherung bzw. Abtrennung des Acroleins deutlich einfacher möglich, in dem überwiegend die an Acrolein hochkonzentrierte Acroleinreaktionsgasphase aus dem Acroleinreaktionsbereich durch eine entsprechende Druckregelung ausgetragen werden kann und anschließend durch Entspannen in hoher Konzentration Acrolein erhalten werden kann.

Je reiner das so erhaltene Acrolein ist, umso weniger ist es notwendig, dass zusätzlich zu dem Entspannen, das beispielsweise über ein als Druckregelventil ausgebildeten Druckregler erfolgen kann, eine Abkühlung über einen Wärmetauscher und eine weitere Auftrennung, die in der Regel destillativ erfolgt, in einer Trenneinheit notwendig ist. Es ist ferner möglich, dass die den Acroleinreaktionsbereich verlassende Acroleinphase über mehrere hintereinander geschaltete, aus einem Überstromventil und einem Wärmetauscher bestehende Einheiten geleitet wird, bevor die so behandelte Acroleinphase einer Trenneinheit zugeleitet wird. Der Druckunterschied vor dem Druckregler in dem Acroleinreaktionsbereich und nach dem Druckregler beträgt vorzugsweise mindestens 30, bevorzugt mindestens 60 und darüber hinaus bevorzugt mindestens 100 bar. Weiterhin ist es in den erfindungsgemäßen Verfahren bevorzugt, dass das Acrolein in dem Acroleinreaktionsbereich mindestens teilweise im überkritischen Zustand vorliegen kann, was zur Ausbeutesteigerung beiträgt.

Weiterhin ist es in dem erfindungsgemäßen Verfahren vorteilhaft, dass die Acroleinreaktionsphase vor dem Abreichern gemäß dem Verfahrensschritt (b) unter einem höheren Druck als beim Abreichern steht. Auch ist es erfindungsgemäß bevorzugt, dass die Acroleinkonzentration in der Acroleinreaktionsphase vor dem Abreichern gemäß dem Verfahrensschritt (b) um mindestens 5, vorzugsweise mindestens 10 und besonders bevorzugt mindestens 50 % höher ist als nach dem Abreichern. Ferner ist es in den erfindungsgemäßen Verfahren bevorzugt, dass ein Schleppgas eingesetzt wird. Dieses Schleppgas wird vorzugsweise vor dem Acroleinreaktionsbereich eingespeist und dient zum Austrag der Acroleinreaktionsphase. Auch in diesem Zusammenhang ist es vorteilhaft, möglichst viel Acrolein in einem gasförmigen Teil der Acroleinreaktionsphase vorzufinden. Als Schleppgase kommen grundsätzlich alle dem Fachmann gegenüber den an den vorstehenden Verfahren beteiligten Verbindungen inerten Gase in Betracht. Beispiele für derartige Schleppgase sind Stickstoff, Luft, CO₂, Wasser oder Argon. Es ist somit in dem erfindungsgemäßen Verfahren bevorzugt, dass das Schleppgas mindestens teilweise nach Durchlaufen des Acroleinreaktionsbereichs wieder in den Acroleinreaktionsbereich eingespeist wird. Diese Einspeisung kann unmittelbar vor dem Acroleinreaktionsbereich oder auch an jeder anderen Stelle vor dem Acroleinreaktionsbereich erfolgen und kann beispielsweise dazu genutzt werden, um einen Vordruck der Edukte aufzubauen, der durch eine entsprechende Pumpe zu den für den Acroleinreaktionsbereich notwendigen Druckverhältnissen weiter verdichtet wird.

Im Verfahrensschritt b) des erfindungsgemäßen Verfahrens zur Herstellung von Acrolein erfolgt ein mindestens teilweises Auftrennen der wässrigen Acroleinreaktionsphase in eine Acrolein-reiche Acroleinphase und eine im Vergleich zu der Acroleinphase Acrolein-arme Restphase R1, wobei die Restphase R1 Glycerin, Wasser und von Glycerin und Wasser verschiedene Reststoffe beinhaltet, wobei dieses Auftrennen vorzugsweise über eine Destillation erfolgt, bei der die Restphase R1 vorzugsweise als Sumpfprodukt erhalten wird.

Im Verfahrensschritt (c) des erfindungsgemäßen Verfahrens zur Herstellung von Acrolein wird zumindest eines Teils der Restphase R1 in Schritt (a)zurückgeführt.

Das erfindungsgemäße Verfahren zur Herstellung von Acrolein sieht nun vor, dass
i) aus einer Glycerinphase G2 beinhaltend Glycerin, Wasser und von Glycerin und Wasser verschiedene Reststoffe mindestens einer dieser Reststoffe abgetrennt und die so erhaltene, aufgereinigte Glycerinphase G2 unmittelbar dem Acroleinreaktionsbereich zugeführt wird, oder
ii) aus einer Mischphase M1, erhalten durch das Vermischen einer Glycerin-phase G2, beinhaltend Glycerin, Wasser und von Glycerin und Wasser verschiedene Reststoffe, mit der Acrolein-armen Restphase R1, mindestens einer der von Glycerin und Wasser verschiedenen Reststoffe abgetrennt und die so erhaltene, aufgereinigte Mischphase M1 unmittelbar dem Acroleinreaktionsbereich zugeführt wird.

Die Formulierung "*wobei die aufgereinigte Glycerinphase G2 unmittelbar dem Acroleinreaktionsbereich zugeführt wird*", wie sie im Zusammenhang mit der Alternative i) des erfindungsgemäßen Verfahrens zur Herstellung von Acrolein verwendet wird, soll zum Ausdruck bringen, dass die aufgereinigte Glycerinphase ohne weitere Aufreinigung dem Acroleinreaktionsbereich zugeführt wird. Dieses schließt jedoch nicht aus, dass der aufgereinigten Glycerinphase G2 vor dem Eintritt in den Acroleinreaktionsbereich weitere Stoffströme, beispielsweise eine weitere Glycerinphase, insbesondere beispielsweise technisches Glycerin, zugesetzt werden. Entsprechendes gilt auch für die Formulierung "*wobei die so erhaltene*, *aufgereinigte Mischphase M1 unmittelbar dem Acroleinreaktionsbereich zugeführt wird*" im Zusammenhang mit der Alternative ii) des erfindungsgemäßen Verfahrens zur Herstellung von Acrolein.

Erfindungsgemäß besonders bevorzugt ist es, dass es sich bei der Glycerinphase G2 um eine Rohglycerinphase handelt, welche als ein Reaktionsprodukt bei der Spaltung oder der Umesterung von Triglyceriden, etwa im Verlaufe der Biodieselherstellung, erhalten wird.

Die Glycerinphase G2, vorzugsweise das vorstehend beschriebe Rohglycerin, beinhaltet üblicherweise, neben dem Glycerin und Wasser, weitere Reststoffe, insbesondere aus der Gruppe bestehend aus Salzen, Fettsäuren, Seifen, Monoglyceriden, Diglyceriden, Triglyceriden und Kondensationsprodukten des Glycerins. Der Wasseranteil im Rohglycerin liegt üblicherweise in einem Bereich von etwa 5 bis etwa 25 Gew.-%, insbesondere in einem Bereich von etwa 15 bis 20 Gew.-%, jeweils bezogen auf die Glycerinphase G2, vorzugsweise das Rohglycerin. Der Anteil von Glycerin liegt üblicherweise zwischen 70 und 90 Gew.-%, insbesondere zwischen 75 und 85 Gew.-%, jeweils bezogen auf die Glycerinphase G2, vorzugsweise das Rohglycerin. Die Salze sind insbesondere Alkalisalze von anorganischen und organischen Säuren. Es handelt sich insbesondere um Natrium- und Kaliumssalze. Die Salze sind üblicherweise solche der Salz-, Schwefel- Phosphor- oder Citronensäure. Zu beachten ist, dass die jeweilige Zusammensetzung einer Glycerinphase G2, vorzugsweise einer Rohglycerinphase, zum einen davon abhängig ist, ob sie aus der Fettspaltung oder der Fettumesterung (also beispielsweise der Biodieselherstellung) stammt und zum anderen davon, wie hoch die Reinheit der zur Fettspaltung bzw. Fettumesterung eingesetzten tierischen oder pflanzlichen Öle bzw. Fette ist.

Im Zusammenhang mit einer solchen Rohglycerinphase als Glycerinphase G2 bedeutet das Abtrennen von Reststoffen aus der Glycerinphase G2 bzw. aus der Mischphase M1, dass der Anteil mindestens eines Reststoffes aus diesen Zusammensetzungen um mindestens 10%, bevorzugt mindestens 20%, weiter bevorzugt mindestens 50%, und besonders bevorzugt mindestens 80 oder 90% reduziert wird. Dies betrifft insbesondere den Anteil der Salze oder einzelner Ionen, insbesondere der Chlorid-Ionen. In einer bevorzugten Ausführungsform der Erfindung wird der Anteil an Chlorid-Ionen in der Glycerin-Phase G2 bzw. in der Mischphase M1 derart vermindert, dass der Anteil an Chlorid-Ionen in der Acroleinreaktionsphase < 50 ppm, bevorzugt < 20 ppm, besonders bevorzugt < 5 oder < 10 ppm beträgt. Vorzugsweise weist die Glycerinphase G2 und/oder die Mischphase M1 nach dem Abtrennen der Reststoffe eine solche Reinheit auf, dass eine verminderte oder im Wesentlichen keine Korrosion der Vorrichtung mehr stattfindet und/oder die Synthese des Acroleins nicht oder weniger beeinträchtigt wird.

In einer besonderen Ausführungsform der Erfindung erfolgt die Herstellung des Rohglycerins durch Spaltung von Glyceriden und die anschließende Weiterverarbeitung des Glycerins zu Acrolein in einem einzigen kontinuierlichen Prozess. Es ist jedoch auch möglich, das Rohglycerin gesondert zu erzeugen und in das erfindungsgemäße Verfahren einzubringen.

Das Abtrennen von Reststoffen gemäß den Alternativen i) oder ii) erfolgt vorzugsweise durch mindestens eine, besonders bevorzugt mindestens zwei und noch mehr bevorzugt jede der folgenden Abtrennarten:
(α1) Destillation;
(α2) Filtration, vorzugsweise ausgewählt aus der Gruppe bestehend aus einer Mikrofiltration, einer Ultrafiltration, einer Umkehrosmose, einer Nanofiltration oder mindestens zwei davon;
(α3) Elektrodialyse;
(α4) Ionenaustauscherbehandlung;
(α5) Behandlung mit Aktivkohle.

Grundsätzlich können die vorstehenden Abtrennarten in jeder dem Fachmann bekannten und geeignet erscheinenden Reihenfolge kombiniert werden. Ferner ist es möglich, auch die gleiche Abtrennart zwei oder mehrfach zu kombinieren, wobei vorzugsweise zwischen den gleichen Abtrennarten mindestens eine von dieser Abtrennart verschiedene Abtrennart vorgesehen ist. So gilt, dass jede Abtrennart für sich eine Ausgestaltung des erfindungsgemäßen Verfahrens darstellen kann. Die durch nachfolgende Ziffernfolgen dargestellten Kombinationen und Abfolgen der Abtrennarten stellen gleichfalls jeweils eine Ausgestaltung des erfindungsgemäßen Verfahrens dar: α1α2, α1α3, α1α4, α2α3, α2α4, α3α4, α2α5. Erfindungsgemäß besonders bevorzugt ist es, dass die Abtrennung durch Nanofiltration, Umkehrosmose oder beides erfolgt. Weiterhin vorteilhaft kann eine Abtrennung mittels Nanofiltration, gefolgt von einer Behandlung mit Aktivkohle sein. Die vorstehend genannten Abtrennverfahren sollen es insbesondere ermöglichen, bei einem Einsatz von Rohglycerin als Glycerinphase G2 dem Rohglycerin mindestens teilweise, vorzugsweise mindestens 10 Gew.-% und besonders bevorzugt mindestens 60 Gew.-% der Begleitsalze zu entziehen.

Erfolgt die Abtrennung mittels Destillation (α1), so kann diese zwei- oder mehrstufig erfolgen. Hierbei ist es bevorzugt, dass die zweite oder jede weitere Stufe bei einem geringeren Druck als die erste Stufe erfolgt. Vorzugsweise wird die zweite bzw. weitere Stufe bei einem Druck in einem Bereich von 20 und 400 mbar, bevorzugt in einem Bereich von 30 bis 300 mbar und besonders bevorzugt in einem Bereich von 50 bis 150 mbar durchgeführt. Die erste Stufe erfolgt meistens bei einem Druck in einem Bereich von 500 mbar bar 1 bar.

Weiterhin kann die Abtrennung mittels Filtration (a2) erfolgen. In diesem Zusammenhang ist es gemäß einer besonders bevorzugten Ausgestaltung des erfindungsgemäßen Verfahrens zur Herstellung von Acrolein, bei der Rohglycerin aus der Fettspaltung oder der Fettumesterung als Glycerinphase G2 eingesetzt wird, bevorzugt, dass das Abtrennen gemäß den Verfahrensvarianten i) und ii) durch Nanofiltration, Umkehrosmose oder durch eine Kombination dieser Verfahren, besonders bevorzugt jedoch durch Nanofiltration erfolgt. Überraschenderweise wurde nämlich festgestellt, dass sie wässrige Glycerinlösungen, wie beispielsweise Rohglycerin, im Gegensatz zu reinem Glycerin mit sehr hohem Permeatfluss mittels Nanofiltration reinigen lassen, wobei ein beachtlicher Teil der im Rohglycerin enthaltenen Salzfracht abgetrennt werden kann.

Die Nanofiltration (NF) ist ein Membrantrennverfahren, welches sich zwischen den Trenngrenzen der Umkehrosmose und der Ultrafiltration einfügt. Der Betriebsdruck liegt bevorzugt im Bereich zwischen 0,3 und 4,0 MPa. Die Ausschlussgrenzen liegen bevorzugt zwischen 100 und 3000 Dalton, besonders bevorzugt zwischen 180 und 2000 Dalton. Die Selektivität einer NF-Membran wird hauptsächlich durch zwei unterschiedliche Parameter bestimmt. Zunächst ist der Rückhalt von der Größe der Verbindungen, also vom Molekulargewicht abhängig. Der Rückhalt und die Permeabilität sind für NF Membranen jedoch auch wesentlich von der elektrischen Ladung und Wertigkeit der Salze und Verbindungen in Lösung abhängig. Verdünnte Lösungen von einwertigen Ionen können eine NF Membran weitgehend ungehindert passieren, während mehrwertige Ionen (wie Sulfat und Carbonat) zu einem großen Teil zurückgehalten werden.

Vorzugsweise erfolgt die Nanofiltration mittels einer polymeren Nanofiltrationsmembran, welche vorzugsweise in einem sogenannten Wickelmodul enthalten ist.

Nach dem Crossflow-Prinzip zu verwendende Wickelmodule sind zum Beispiel aus der DE 43 28 407 C1 bekannt. Sie bestehen aus einem Gehäuse mit einem darin eingeschlossenen Wickel. In einem solchen Wickelmodul haben die Membranen die Form von dreiseitig verschweißten Taschen mit einem porösen Membranträger im Innern der Tasche. Sie sind gemeinsam mit einem Trenngeflecht (*Feed-Spacer*) um ein perforiertes Zentralrohr gewickelt. Die zu filtrierende Lösung, beispielsweise das zu filtrierende Rohglycerin (*Feed*), durchströmt unter hohem Druck in axialer Richtung den Spalt zwischen den Membrantaschen, wobei ein Teil des Wassers und des Glycerins durch die Membran in die Taschen permeiert und im Membranträger spiralförmig dem zentralen Sammelrohr zufließt. Durch die nicht verschweißte Seite der Tasche, die mit dem Zentralrohr verbunden ist, tritt das Permeat in selbiges ein und wird von dort aus dem Modul geleitet.

Im Zusammenhang mit einem derartigen Wickelmodul ist es weiterhin vorteilhaft, wenn dieses einen Feedspacer von mindestens 40 mm, besonders bevorzugt von mindestens 45 mm und am meisten bevorzugt von mindestens 50 mm aufweist.

Als Nanofiltrationsmembran kommen erfindungsgemäß insbesondere polymere Nanofiltrationsmembran, welche aus einem Polyamid oder einem Polyethersulfon gefertigt ist, in Betracht. Neben diesen Membrantypen können jedoch grundsätzlich auch all diejenigen Nanofiltrationsmembranen eingesetzt werden, die dem Fachmann aus dem Stand der Technik bekannt sind. Weiterhin zu nennen sind hier insbesondere Membranen auf der Basis von Polysulfon, Polyacrylnitril, Polyethylen, Teflon, porösem Kohlenstoff, Keramik, Celluloseacetat, Polyharnstoff, aromatischen oder aliphatischen Polyamiden, sulfonierten Polyarylethern, Polyfuran, Polybenzimidazol, verschiedenen Fluor-polymeren, Polyetheraromaten wie Polyimid oder Polyimidazopyrrolidon oder ähnlichen Materialien. Erfindungsgemäß als Nanofiltrationsmembranen geeignet sind insbesondere die unter der Bezeichnung "Nadir N30F" von der Firma Microdyn-Nadir GmbH und unter den Bezeichnungen "DESAL 5DK" bzw. "DESAL 5 DL" von unter der Firma GE Osmonics, USA erhältlichen Membranen.

Erfindungsgemäß besonders vorteilhaft ist der Einsatz von Nanofiltrationsmembranen mit einer Ausschlussgröße von 2.000 kDa oder weniger, besonders bevorzugt von 1.500 kDa oder weniger, noch mehr bevorzugt von 1.000 kDa oder weniger und am meisten bevorzugt von 500 kDa oder weniger.

Im Zusammenhang mit dem Abtrennen mittels Nanofiltration ist es weiterhin bevorzugt, dass das Abtrennen mit einer Permeat-Ausbeute von mindestens 90 Gew.-%, besonders bevorzugt mindestens 95 Gew.-% und am meisten bevorzugt mindestens 99 Gew.-% durchgeführt wird.

Weiterhin ist es im Zusammenhang mit dem Abtrennen mittels Nanofiltration bevorzugt, dass dieses Abtrennen bei einer Temperatur von vorzugsweise mehr als 40°C, besonders bevorzugt mehr als 45°C und am meisten bevorzugt mehr als 50°C und bei einem Transmembrandruck von vorzugsweise mehr als 30 bar, besonders bevorzugt mehr als 40 bar und am meisten bevorzugt mehr als 50 bar erfolgt.

Bei der Umkehrosmose (reverse Osmose) benutzt man Druck, um den natürlichen Osmose-Prozess umzukehren. Das Medium, in dem die Konzentration eines bestimmten Stoffes verringert werden soll, ist durch eine halbdurchlässige (semipermeable) Membran von dem Medium getrennt, in dem die Konzentration erhöht werden soll. Das erste Medium wird einem Druck ausgesetzt, der höher sein muss als der Druck, der durch das osmotische Verlangen zum Konzentrationsausgleich entsteht. Dadurch können die Moleküle des Lösungsmittels gegen ihre "natürliche" osmotische Ausbreitungsrichtung in den Bereich wandern, in dem die gelösten Stoffe bereits geringer konzentriert sind. Die Ultrafiltration ist primär ein von der Teilchengröße abhängiger, druckgetriebener Stofftrennprozess auf Grundlage eines Siebeffektes. Ultrafiltrationsmembranen haben bevorzugt eine Porengröße zwischen 0,5 und 200 nm, insbesondere zwischen 1 und 100 nm und sind bevorzugt in der Lage, Verbindungen mit einem Molekulargewicht von mehr als 200 Dalton, insbesondere mehr als 300 Dalton zurückzuhalten.

Neben der erfindungsgemäß am meisten bevorzugten Nanofiltration und neben der Ultrafiltration kann, sofern die Abtrennung mittels Filtration (α2) erfolgt, auch die Mikrofiltration als Abtrennverfahren gemäß den Varianten i) und ii) des erfindungsgemäßen Verfahrens zur Herstellung von Acrolein Verwendung finden. Bei einer Mikrofiltration (MF) wird in einem vorzugsweise druckgetriebenen Prozess eine Membran zur Trennung von Partikeln aus wässrigen Lösungen verwendet. Die Mikrofiltration ist eine Filtration von kolloidalen oder anderen feinteiligen Partikeln mit einer linearen Dimension von etwa 0,02 µm bis etwa 10 µm. Der typische Betriebsdruck einer MF ist verhältnismäßig niedrig und beträgt zwischen 0,02 MPa und 0,5 MPa.

Weiterhin kann die Abtrennung mittels Elektrodialyse (α3) erfolgen. Die Elektrodialyse ist ein Trennprozess, bei welchem Ionen unter dem Einfluss eines elektrischen Potenzials durch eine semipermeable Membran transportiert werden. Die verwendeten Membranen sind nur für positiv oder negativ geladene Ionen durchlässig, d.h. sie arbeiten kationen- oder anionenselektiv. Kationenselektive Membranen sind Polyelektrolyte mit negativ geladener Materie, die negativ geladene Ionen zurückhalten und durchlässig für positiv geladene Ionen sind. Durch die Anordnung einer Vielzahl von Kammern, die abwechselnd von anionen- und kationenselektiven Membranen begrenzt sind, können Ionen aus der Lösung entfernt werden. Die Kammern befinden sich zwischen den Flächenelektroden Anode und Kathode. Durch die Anordnung werden die Ionen der Lösung in einigen Kammern aufkonzentriert, während sie in anderen Kammern die Lösung verlassen können. Die Lösungen mit erhöhter Salzkonzentration werden zum "Konzentrat" vereint, während die salzarmen Lösungen das "Diluat" bilden. Die aufkonzentrierte Lösung zirkuliert, bis eine Endkonzentration erreicht ist und wird im Anschluss abgeschlagen. Dieses Verfahren eignet sich besonders zur Ionenentfernung aus wässrigen Lösungen. Ungeladene Partikel lassen sich dagegen nicht eliminieren. Kationenselektive Membranen bestehen bevorzugt aus sulfoniertem Polystyrol, während anionenselektive Membranen bevorzugt aus Polystyrol mit quaternären Aminen aufgebaut sind. Teilweise ist vor dem Elektrodialyse-Verfahren eine Vorbehandlung der Lösung notwendig. In einer besonderen Ausführungsform werden suspendierte Feststoffe, insbesondere mit einem Durchmesser über 10 mm, vorher entfernt, um ein Verblocken der Membranporen zu vermeiden. Bei der Elektrodialyse kann eine Umkehr der Fließrichtung vorgesehen sein. Dadurch kann eine regelmäßige Reinigung von Belägen erreicht werden.

Denkbar ist weiterhin auch eine Abtrennung mittels einer Ionenaustauscherbehandlung (α4). Eine Ionenaustauscherbehandlung erfolgt an Vorrichtungen, mit denen im Wasser gelöste Ionen gegen andere Ionen ausgetauscht werden. Dabei handelt es sich beispielsweise um Säulen, die mit einem Ionenaustauschermaterial, dem Ionenaustauscherharz, gefüllt sind, und von der zu behandelnden Lösung durchströmt werden. Die zu ersetzenden Ionen werden am Ionenaustauscher-Material gebunden, das seinerseits dabei Ionen in die Lösung abgibt. Erfindungsgemäß werden bevorzugt Salz-Ionen der Lösung durch Protonen und Hydroxy-Ionen ersetzt, so dass die Lösung insgesamt entsalzt wird. In speziellen Ausführungsformen der Erfindung ist die erfindungsgemäße Ionenaustauscherbehandlung keine Elektrodialyse, es wird keine elektrische Spannung angelegt und/oder kein erhöhter Druck verwendet.

Gemäß einer besonderen Ausgestaltung des erfindungsgemäßen Verfahrens erfolgt die Abtrennung der von Wasser und Glycerin verschiedenen Reststoffe gemäß den Verfahrensvarianten i) oder ii) durch eine Filtration, besonders bevorzugt durch eine Nanofiltration oder eine Umkehrosmose, am meisten bevorzugt durch eine Nanofiltration, gefolgt von einer Elektrodialyse. In diesem Zusammenhang kann es weiterhin vorteilhaft sein, wenn auf die Elektrodialyse eine osmotische Reinigung, vorzugsweise eine weitere Reinigung durch Umkehrosmose, folgt, wobei sich der Elektrodialyse oder Umkehrosmose wiederum eine Ionenaustauscherbehandlung anschließend kann.

Gemäß einer anderen besonderen Ausgestaltung des erfindungsgemäßen Verfahrens erfolgt die Abtrennung der von Wasser und Glycerin verschiedenen Reststoffe gemäß den Verfahrensvarianten i) oder ii) zunächst durch eine Filtration, besonders bevorzugt durch eine Mikrofiltration, eine Nanofiltration oder eine Umkehrosmose, am meisten bevorzugt durch eine Mikrofiltration, gefolgt von einer Elektrodialyse, gefolgt von einer weiteren Filtration, besonders bevorzugt einer Nanofiltration oder einer Umkehrosmose und wiederum gefolgt von einer Ionenaustauscherbehandlung.

In einer weiteren bevorzugten Ausführungsform der Erfindung wird zur Abtrennung von Reststoffen aus der Glycerinphase G2, vorzugsweise aus dem Rohglycerin, bzw. aus der Mischphase M1 ein integriertes Membransystem zur Entsalzung verwendet, bei dem elektrische Trennverfahren mit Trennungsschritten an Membranen unter erhöhtem Druck kombiniert werden. Dabei wird eine Elektrodialyse mit einer Nanofiltration oder Reversen Osmose (Umkehrosmose) kombiniert. Entsprechende Vorrichtungen werden in der WO-A- 2006/074259 beschrieben, auf die hier ausdrücklich Bezug genommen wird. Gegenstand der Erfindung ist in einer bevorzugten Ausführungsform ein erfindungsgemäßes Verfahren zur Herstellung von Acrolein, bei dem die Reinigung einer glycerinhaltigen Lösung mit einer Vorrichtung gemäß WO-A-2006/074259 erfolgt. Es ist überraschend, dass eine solche Vorrichtung auch zur Reinigung von hoch konzentrierten Glycerinlösungen verwendet werden kann.

In dem erfindungsgemäßen Verfahren zur Herstellung von Acrolein wird neben der aufgereinigten Glycerinphase G2 oder der aufgereinigten Mischphase M1 eine weitere Glycerinphase G3 dem Acroleinreaktionsbereich zugeführt, wobei die Glycerinphase G3 einen höheren Glyceringehalt als die Glycerinphase G2 und einen geringeren Salzgehalt als die Glycerinphase G2 und einen geringeren Gehalt an von Glycerin verschiedenen organischen Verbindungen als die Glycerinphase G2 aufweist. Oftmals unterscheiden sich die Gehalte der Glycerinphasen G2 und G3 im Hinblick auf das Glycerin, das Salz und die von Glycerin verschiedenen organischen Verbindungen um mindestens 1 Gew.-%, vorzugsweise mindestens 2 Gew.-% und besonders mindestens 5 Gew.-%, jeweils bezogen auf die jeweilige Glycerinphase.

Bei der Glycerinphase G3 handelt es sich um das bei der Destillation von bei der Spaltung oder der Umesterung von Triglyceriden erhaltenem Rohglycerin anfallende Destillat. Dieses Destillat wird herkömmlich als "technisches Glycerin" bezeichnet. Technisches Glycerin hat üblicherweise einen Glycerinanteil von mindestens 70 Gew.-%, insbesondere mindestens 80 Gew.-% und vorzugsweise mindestens 90 Gew.-%. Der Wasseranteil beträgt üblicherweise bis zu 30 Gew.-%, bevorzugt bis zu 20 Gew.-% und vorzugsweise bis zu 10 Gew.-%. Als von Glycerin und Wasser verschiedene Reststoffe beinhaltet auch die Glycerinphase G3 vorzugsweise Salze, Fettsäuren, Seifen, Monoglyceride, Diglyceride, Triglyceride und Kondensationsprodukte des Glycerins. Die sonst im Rohglycerin anzutreffenden Reststoffe liegen um das mindestens 10-fache niedriger beim technischen Glycerin. Mindestens ein Teil des bei der Destillation des Rohglycerins anfallenden Sumpfprodukts durchläuft einen Salzabscheider und wird anschließend wieder der Destillation des Rohglycerins zugeführt werden, um die Ausbeute an Glycerin bei der Aufreinigung des Rohglycerins weiter zu steigern.

Weiterhin hat es sich bei der Destillation des Rohglycerins bewährt, dass mindestens ein Teil des dabei erhaltenen Destillats, vorzugsweise mindestens 50 Gew.-%, besonders bevorzugt mindestens 75 Gew.-% oder in einem Bereich von 60 bis 100 Gew.-% des Destillats einen Ionenaustauscher durchläuft. Weiterhin kann es sich als vorteilhaft erweisen, die Destillation in einer ersten und mindestens einer weiteren Stufe bei verschiedenen Drücken erfolgen zu lassen, wobei der Druckunterschied mindestens 0,1 bar, vorzugsweise mindestens 1 bar und besonders bevorzugt mindesten 2 bar beträgt. Ferner ist es in dem erfindungsgemäßen Verfahren bevorzugt, die Destillation in einer ersten Stufe bei einem höheren Druck als die Destillation in der mindestens einen weiteren Stufe erfolgen zu lassen.

Als Reststoffe beinhalten die Glycerinphase G3 oder die Glycerinphase G2 oder beide üblicherweise Salze, Fettsäuren, Seifen, Monoglyceride, Diglyceride, Triglyceride und Kondensationsprodukte des Glycerins.
1. In einer ersten besonderen Ausgestaltung des erfindungsgemäßen Verfahrens zur Herstellung von Acrolein ist es bevorzugt, dass es sich bei der Glycerinphase G2 um Rohglycerin handelt, welches beispielsweise aus der Fettspaltung oder der Fettumesterung erhalten wurde, welches der Restphase R1 zugesetzt wird, wobei aus der so erhaltenen Mischphase M1 anschließend durch eine Filtration, besonders bevorzugt durch die vorstehend beschriebene Nanonfiltration, Reststoffe abgetrennt werden. Vor dieser Abtrennung durch Nanofiltration kann die Mischphase M1 zur Abtrennung von in dieser Mischphase enthaltenen Feststoffen gegebenenfalls auch über eine Vorfiltration gereinigt werden. Weiterhin kann bei dieser ersten besonderen Ausführungsform neben dem Einsatz von Rohglycerin in der vorstehend beschriebenen Art und Weise auch technisches Glycerin als weitere Glycerin-phase G3 eingesetzt werden, wobei es in diesem Fall bevorzugt ist, das technische Glycerin entweder direkt dem Acroleinreaktionsbereich zuzuführen oder aber zunächst der aufgereinigte Mischphase M1 zuzusetzen und die so erhaltene Zusammensetzung dann dem Acroleinreaktionsbereich zuzuführen. Diese erste besondere Ausgestaltung des erfindungsgemäßen Verfahrens zur Herstellung von Acrolein verwirklicht somit die Variante ii) des erfindungsgemäßen Verfahrens zur Herstellung von Acrolein. Diese Ausführungsform des erfindungsgemäßen Verfahrens eignet sich insbesondere für einen Einsatz von Rohglycerin mit geringen Chlorid-Gehalten, vorzugsweise mit Chlorid-Gehalten von weniger als 1 g/l.
2. In einer zweiten besonderen Ausgestaltung des erfindungsgemäßen Verfahrens zur Herstellung von Acrolein ist es bevorzugt, dass es sich bei der Glycerinphase G2 um Rohglycerin handelt, welches beispielsweise aus der Fettspaltung oder der Fettumesterung erhalten wurde, welches der Restphase R1 zugesetzt wird, wobei aus der so erhaltenen Mischphase M1 anschließend durch eine Filtration, besonders bevorzugt durch die vorstehend beschriebene Nanonfiltration, Reststoffe abgetrennt werden. Auf diese Filtration folgt weiterhin eine Aufreinigung mittels Elektrodialyse, welcher gegebenenfalls noch eine Ionenaustauscherbehandlung folgt. Auch bei dieser zweiten besonderen Ausführungsform des erfindungsgemäßen Verfahrens kann es sich als vorteilhaft erweisen, wenn vor der Abtrennung von Reststoffen durch Nanofiltration die Mischphase M1 zur Abtrennung von in dieser Mischphase enthaltenen Feststoffen gegebenenfalls auch über eine Vorfiltration und/oder einen Ionenaustauscher gereinigt wird. Auch bei dieser zweiten besonderen Ausführungsform wird neben dem Einsatz von Rohglycerin in der vorstehend beschriebenen Art und Weise auch technisches Glycerin als weitere Glycerinphase G3 eingesetzt, wobei es in diesem Fall bevorzugt ist, das technische Glycerin entweder direkt dem Acroleinreaktionsbereich zuzuführen oder aber zunächst der aufgereinigte Mischphase M1 zuzusetzen und die so erhaltene Zusammensetzung dann dem Acroleinreaktionsbereich zuzuführen. Diese zweite besondere Ausgestaltung des erfindungsgemäßen Verfahrens zur Herstellung von Acrolein verwirklicht somit ebenfalls die Variante ii) des erfindungsgemäßen Verfahrens zur Herstellung von Acrolein. Diese Ausführungsform des erfindungsgemäßen Verfahrens eignet sich insbesondere für einen Einsatz von Rohglycerin mit höheren Chlorid-Gehalten, vorzugsweise mit Chlorid-Gehalten von mehr als 1 g/l.
3. In einer dritten besonderen Ausgestaltung des erfindungsgemäßen Verfahrens zur Herstellung von Acrolein ist es bevorzugt, dass es sich bei der Glycerinphase G2 um Rohglycerin handelt, welches beispielsweise aus der Fettspaltung oder der Fettumesterung erhalten wurde, wobei aus diesem Rohglycerin zunächst Reststoffe durch Destillation abgetrennt werden, gefolgt von einer weiteren Abtrennung von Reststoffen durch einen Ionenaustauscher, wobei die so erhaltene, aufgereinigte Glycerinphase G2 nach Kombination mit einer weiteren Glycerinphase, insbesondere nach Kombination mit technischem Glycerin, sowie gegebenenfalls nach Kombination mit aufgereinigter Restphase R1, dem Acroleinreaktionsbereich zugeführt wird. Der bei der Destillation des Rohglycerins anfallende Rückstand kann mit einem Salzabscheider behandelt werden. Neben der Abtrennung von Reststoffen aus der Glycerinphase G2 durch die Kombination aus Destillation und Ionenaustausch kann die Aufreinigung auch durch Filtration, insbesondere durch die vorstehend beschriebene Nanonfiltration, erfolgen. Aus der im Verfahrensschritt b) nach dem Auftrennen der wässrigen Acroleinreaktionsphase erhaltenen Restphase R1 werden anschließend ebenfalls Reststoffe unter Erhalt einer aufgereinigten Restphase R1 abgetrennt, wobei dieses Abtrennen vorzugsweise durch eine Filtration, besonders bevorzugt durch die vorstehend beschriebene Nanonfiltration, erfolgt. Auch hier kann es sich als vorteilhaft erweisen, vor dieser Abtrennung durch Nanofiltration die Mischphase M1 über eine Vorfiltration zur Abtrennung von in dieser Mischphase enthaltenen Feststoffen aufzureinigen. Die aufgereinigte Restphase R1 kann dann unmittelbar dem Acroleinreaktionsbereich zugeführt oder zunächst der aufgereinigten Rohglycerinphase zugesetzt werden, wie vorstehend beschrieben. Diese dritte besondere Ausgestaltung des erfindungsgemäßen Verfahrens zur Herstellung von Acrolein verwirklicht somit die Variante i) des erfindungsgemäßen Verfahrens zur Herstellung von Acrolein.
Bei dieser dritten besonderen Ausführungsform des erfindungsgemäßen Verfahrens zur Herstellung von Acrolein kann mindestens ein Teil der erfindungsgemäß gereinigten Glycerinlösung aus dem Prozess entnommen werden, bevor diese zurück in den Acroleinreaktionsbereich überführt wird. Bevorzugt hat das gereinigte Glycerin die Qualität technischen Glycerins. Die so erhaltene gereinigte Glycerinlösung kann für andere Anwendungen, beispielsweise für andere Syntheseprozesse, verwendet werden.

Allgemein weisen die beschriebenen Verfahren den Vorteil auf, dass nach der Entfernung des Acroleins in der Destillationseinheit und der Abtrennung der abgereicherten Reaktionsphase (Verfahrenschritt b)) die weitere Reinigung erfolgt, ohne dass eine weitere Destillation erforderlich ist. Die Reinigungsmaßnahmen sind vergleichsweise kosten- und energiearm und schonend für die jeweiligen Glycerin-haltigen Lösungen aufgrund der vergleichsweise niedrigen Temperaturen, die zum Einsatz kommen. Durch die Kombination dieser Reinigungsschritte wird schrittweise die Entsalzung der glycerinhaltigen Lösung erreicht.

Ein weiterer Vorteil ist, dass das erhaltene aufgereinigte Glycerin eine gute Farbzahl aufweist, da es nicht thermisch belastet wurde. Durch die spezielle erfindungsgemäße Kombination der Reinigungsschritte wird außerdem eine hohe Reinheit erreicht. Das Verfahren ist insbesondere zur Aufreinigung von Rohglycerin mit hohen Anteilen von Salzen, Chloriden und organischen Substanzen geeignet.

Weiterhin entspricht es einer Ausgestaltung des erfindungsgemäßen Verfahrens, dass ein Teil, vorzugsweise mindestens 5 Gew.-%, bevorzugt mindestens 35 Gew.-% und noch mehr bevorzugt mindestens 50 Gew.-%, oder auch 15 bis 35 Gew.-%, des Glycerins der Glycerinphase G1 als Zustrom zur Dehydratisierung in dem Verfahren zur Herstellung von Acrolein in einem Kreislauf geführt wird. Hierdurch kann zum einen durch Wahl der richtigen Strömungsgeschwindigkeiten das Entstehen von Ablagerungen verlangsamt oder gar verhindert werden und zum anderen eine genaue Kontrolle der Reaktion erfolgen.

Allgemein wird bei der Rückführung im Zusammenhang mit der Kreislauffahrweise der rückgeführte Strom so eingestellt, dass hohe Acroleinausbeuten bei möglichst hohen Umsätzen erhalten werden. Vorzugsweise liegt das Rücklaufverhältnis der Glycerinphase zu der rückgeführten Acrolein-armen Restphase R1 im Bereich von 0,01 : 10 bis 9 : 10, bevorzugt von 0,1 : 10 bis 5 : 10 und besonders bevorzugt von 0,5 : 10 bis 3 : 10. Die Rückführung dient insbesondere der optimalen Verwertung der Edukte, der Einsparung von Kosten und dem Umweltschutz.

In dem erfindungsgemäßen Verfahren zur Herstellung von Acrylsäure ist es bevorzugt, dass die Acroleinphase in Schritt (A2) in einem Bereich von 0,1 bis 50, bevorzugt 5 bis 30 Gew.-%, vorzugsweise von 7 bis 20 Gew.-% und darüber hinaus bevorzugt von 10 bis 20 Gew.-%, jeweils bezogen auf die Acroleinphase, Acrolein aufweist. Im Zusammenhang mit einer möglichst langen Laufzeit des Oxidationsreaktors ist es bevorzugt, dass die Acroleinphase weniger als 10 Gew.-%, vorzugsweise weniger als 5 Gew.-% und besonders bevorzugt weniger als 2 Gew.-% Bestandteile aufweist, die allgemein als Schwersieder bezeichnet werden, und einen höheren Siedepunkt als Acrolein besitzen. Es ist zudem bevorzugt, dass die Acroleinphase auch weniger als 10 Gew.-%, bevorzugt weniger als 5 Gew.-% und besonders bevorzugt weniger als 2 Gew.-%, jeweils bezogen auf die Acroleinphase, Leichtsieder, d.h. Stoffe die einen geringeren Siedepunkt als Acrolein aufweisen, enthält. Ferner ist es bevorzugt, dass die Acroleinphase neben Acrolein und gegebenenfalls vorhandenen Leicht- bzw. Schwersiedern im Wesentlichen inerte Bestandteile, insbesondere gasförmige Bestandteile, aufweist, die die Oxidationsreaktion in Schritt (A2), wenn überhaupt, nur unwesentlich beeinträchtigen.

Außerdem ist es in dem erfindungsgemäßen Verfahren zur Herstellung von Acrylsäure bevorzugt, dass bei der Oxidation in Schritt (A2) eine Acrylsäure beinhaltende, gasförmige Acrylsäurephase entsteht, wobei aus dieser Acrylsäurephase Acrylsäure abgereichert wird und mindestens ein Teil der abgereicherten Acrylsäurephase in den Schritt (A2) eingespeist wird. Hierbei ist es bevorzugt, dass der Teil der abgereicherten Acrylsäurephase vor dem Einspeisen einer Verbrennung, vorzugsweise einer Gasphasenverbrennung und besonders bevorzugt einer katalytischen Gasphasenverbrennung, wie in WO-A-03/051809 beschrieben, unterzogen wird. Eine abgereicherte Acrylsäurephase weist vorzugsweise weniger als 5 Gew.-%, bevorzugt weniger als 1 Gew.-% und darüber hinaus bevorzugt weniger als 0,1 Gew.-%, jeweils bezogen auf die abgereicherte Acrylsäurephase, Acrylsäure auf. Weitere Bestandteile der abgereicherten Acrylsäurephase sind Wasser, Stickstoff und CO₂. Vorteilhafterweise kann der Teil der abgereicherten Acrylsäurephase, insbesondere nach der Verbrennung, als Schleppgas in dem erfindungsgemäßen Verfahren zur Herstellung von Acrylsäure eingesetzt werden. Weiterhin kann der für eine Oxidation des Acroleins benötigte Sauerstoff- bzw. Luftstrom entweder unter gleichzeitiger Verwendung als Schleppgas bei Schritt (a) oder zum Zweck der Oxidation zu Acrylsäure direkt in Schritt (A2) eingespeist werden.

Das erfindungsgemäße Verfahren zur Herstellung von Acrylsäure wird vorzugsweise unter Einsatz einer Vorrichtung zur Herstellung von Acrolein durchgeführt, beinhaltend fluidleitend miteinander verbunden
- einen Reaktor zur Umsetzung von Glycerin in einer wässrigen Glycerinphase zu Acrolein,
- Mittel zur Zuführung einer wässrigen Glycerinphase zu dem Reaktor,
- eine Destillationseinheit zur Abreicherung von Acrolein,
- Abtrennungsmittel zur Abtrennung der abgereicherten Acroleinphase,
- Reinigungsmittel zur Reinigung der abgereicherten Acroleinphase, und
- eine Rückführungsleitung, die die Abtrennungsmittel, die Reinigungsmittel und den Reaktor fluidleitend miteinander verbindet.

Nach einer besonderen Ausgestaltung der Vorrichtung zur Herstellung von Acrolein weist der Reaktor zur Umsetzung von Glycerin zu Acrolein einen Dehydratisierungskatalysator auf. Dieser Dehydratisierungskatalysator ist vorzugsweise fest in dem Reaktor angeordnet. Dieses kann einmal dadurch erreicht werden, in dem der Dehydratisierungskatalysator an Wandungen des Reaktors immobilisiert ist oder, sofern der Dehydratisierungskatalysator in Form von Partikeln vorliegt oder darauf immobilisiert ist, geeignete Siebe- und Filter im Reaktor das Ausschwemmen dieser Partikel verhindern.

Auf den Reaktor folgt vorzugsweise eine Neutralisierungseinheit, aus der Lauge eingespeist wird, bevor die Acroleinreaktionsphase destilliert wird.

In weiteren bevorzugten Ausführungsformen der Vorrichtung zur Herstellung von Acrolein sind die Reinigungsmittel ausgewählt aus einer Filtrationseinheit, Mitteln zur Umkehrosmose, einer Elektrodialyseeinheit, einer Ionenaustauschereinheit oder einer Kombination aus mindestens zwei davon.

Die Filtrationseinheit dient insbesondere zur Ausschleusung von Hochsiedern und umfasst vorzugsweise mindestens einen Mikrofilter, Ultrafilter, einen Nanofilter oder eine Kombination aus mindestens zwei davon, wobei der Nanofilter bevorzugt eine Membran ist.

Gemäß einer bevorzugten Ausführungsform der Vorrichtung zur Herstellung von Acrolein beinhaltet das Reinigungsmittel daher einen Nanofilter, wobei dieser Nanofilter vorzugsweise eine polymere Nanofiltrationsmembran, welche wiederum vorzugsweise aus einem Polyamid oder einem Polyethersulfon gefertigt ist. Als polymere Nanofiltrationsmembranen sind dabei diejenigen polymeren Nanofiltrationsmembranen bevorzugt, die bereits eingangs im Zusammenhang mit dem erfindungsgemäßen Verfahren zur Herstellung von Acrolein als bevorzugte polymere Nanofiltrationsmembranen genannt wurden.

Weiterhin ist es auch im Zusammenhang mit der Vorrichtung zur Herstellung von Acrolein bevorzugt, dass der Nanofilter aus einem die polymere Nanofiltrationsmembran beinhaltenden Wickelmodul gefertigt ist und dass das Wickelmodul einen Feedspacer von mindestens 40 mm aufweist. In diesem Zusammenhang wird ebenfalls auf die entsprechenden Ausführungen zu bevorzugten Wickelmodulen bezüglich des erfindungsgemäßen Verfahrens zur Herstellung von Acrolein verwiesen.

In einer bevorzugten Ausführungsform der Vorrichtung zur Herstellung von Acrolein weist die Filtrationseinheit einen vorgeschalteten Filter zur Abtrennung von festen Bestandteilen, gefolgt von einem Nanofiltrationsfilter und/oder Mitteln zur Umkehrosmose auf.

In einer weiteren bevorzugten Ausführungsform der Vorrichtung zur Herstellung von Acrolein enthalten die Reinigungsmittel eine Filtrationseinheit und/oder Mittel zur Umkehrosmose und eine, bevorzugt nachgeschaltete, Elektrodialyseeinheit. Daran anschließend können Mittel zur Nanofiltration und/oder Umkehrosmose und/oder Ionenaustauscherbehandlung folgen. In einer bevorzugten Ausführungsform ist eine der Elektrodialyseeinheit nachgeschaltete Ionenaustauschereinheit enthalten.

Die Vorrichtung enthält vorzugsweise Mittel zur Reinigung der wässrigen Glycerinphase vor der ersten Einleitung in den Reaktor. Die Mittel zur Reinigung sind dabei bevorzugt ausgewählt aus einer Filtrationseinheit, einer Destillationseinheit, Mitteln zur Umkehrosmose, einer Elektrodialyseeinheit und/oder einer Ionenaustauschereinheit.

Außerdem können Mittel zur Entnahme der abgereicherten Acroleinphase nach der Reinigung durch die Reinigungsmittel enthalten sein.

In einer weiteren bevorzugten Ausführungsform umfasst die Vorrichtung zur Herstellung von Acrolein einen Reaktor zur Erzeugung von Glycerin aus Glyceriden.

In dem erfindungsgemäßen Verfahren kann eine Vorrichtung zur Herstellung von Acrylsäure mit den Merkmalen der Vorrichtung zur Herstellung von Acrolein eingesetzt werden, die zusätzlich einen Oxidationsreaktor zur Oxidation von Acrolein zu Acrylsäure umfasst, wobei die Vorrichtung zur Herstellung von Acrolein fluidleitend mit dem Oxidationsreaktor verbunden ist.

In einer Ausgestaltung weist der Oxidationsreaktor einen Multioxidkatalysator als Pulver, Schicht oder Pellet oder einer Kombination aus mindestens zwei davon auf. Diese Pulver, Schichten oder auch Pellets können sich an Metallwandungen von Metallplatten oder Metallrohren befinden. In der Vorrichtung zur Herstellung von Acrylsäure sind Plattenreaktoren, beispielsweise solche mit Thermoblechen, oder mit mehreren Rohren, auch Rohrbündelreaktoren genannt, bevorzugt, wobei Rohrbündelreaktoren besonders bevorzugt sind. Im Zusammenhang mit der Zusammensetzung der Multioxidkatalysatoren wird auf die Ausführungen in WO-A-03/051809 als Teil dieser Offenbarung Bezug genommen, wobei auf Molybdän, Vanadium und Wolfram basierende Katalysatoren besonders bevorzugt sind. An den Oxidationsreaktor schließt sich vorzugsweise eine Aufarbeitungseinheit an. In diesem Zusammenhang ist es bevorzugt, dass die Aufarbeitungseinheit eine Quencheinheit aufweist. Zudem ist es bevorzugt, dass die Vorrichtung zur Herstellung von Acrylsäure eine Wasserabtrenneinheit aufweist, die vorzugsweise mit der Quencheinheit kombiniert ist und in vorteilhafter Weise zu der Erzeugung der Acrylsäure abgereicherten Acrylsäurephase beiträgt, wobei auch in diesem Zusammenhang wird auf die Offenbarung von WO-A-03/051809 Bezug genommen.

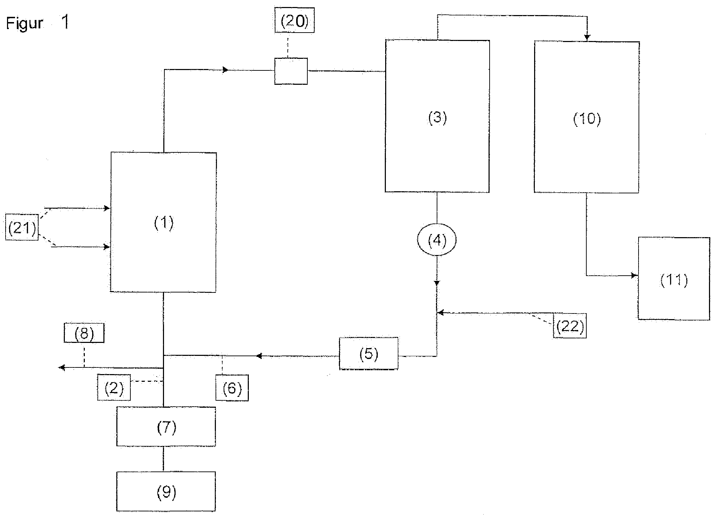

Die Vorrichtung zur Herstellung von Acrylsäure entwickelt beispielsweise, ohne auf die dort offenbarten Ausführungsformen beschränkt zu sein, die in der WO-A-2006/092272 und DE-A-10 2005 028 624 offenbarten Vorrichtungen in erfinderischer Weise weiter, und zwar im wesentlichen hinsichtlich der Ausbildung des Verfahrens als Kreislauf, der Reinigung der abgereicherten Acroleinreaktionsphase, der Aufreinigung des eingespeisten Rohglycerins und der Möglichkeit der Einspeisung von Glycerin in den Kreislauf. Es wird hiermit ausdrücklich auf die in WO-A-2006/092272 und DE-A-10 2005 028 624 offenbarten Vorrichtungen und deren Merkmale Bezug genommen, insbesondere auf die Figuren 1 bis 5 der WO-A-2006/092272 und die entsprechenden Erläuterungen in der Beschreibung sowie auf die Figur 1 der DE-A-10 2005 028 624 und die entsprechenden Erläuterungen in der Beschreibung in Absatz [0061].

Die Vorrichtung zur Herstellung von Acrylsäure ist in einer bevorzugten Ausführungsform durch folgende fluidleitend miteinander verbundene Merkmale charakterisiert:
- eine Dehydratisierungseinheit;
- davon stromabwärts eine Gasphasenoxidationseinheit;
- wobei die Dehydratisierungseinheit
- eine Edukteinspeisung;
- davon stromabwärts einen Acroleinreaktionsbereich;
- davon stromabwärts einen Druckregler; und
- davon stromabwärts eine Abreicherungseinheit aufweist, wobei die Abreicherungseinheit mit der Gasphasenoxidationseinheit fluidleitend verbunden ist;
- sowie erfindungsgemäße Reinigungsmittel (5) und eine Rückführungsleitung (6)
wobei die Gasphasenoxidationseinheit stromabwärts von der Abreicherungseinheit
- einen Reaktor, aufweisend einen Multioxidkatalysator; und
- eine Aufbereitungseinheit aufweist.

Die Mittel zur Zuführung der wässrigen Glycerinphase (Edukteinspeisung) erfolgt in einer Ausführungsform durch Entnahme des Edukts aus einen Tank, der entweder Glycerin als solches oder Glycerin in Form einer wässrigen Lösung aufnehmen kann. Im Zusammenhang mit dem Acroleinreaktionsbereich wird zunächst auf die vorangegangenen Ausführungen Bezug genommen. Außerdem ist es bevorzugt, dass der Acroleinreaktionsbereich in dem Bereich, in dem er rohrförmig ausgebildet ist, eine im Vergleich zum Querschnitt längeren Durchmesser aufweist.

Bei dem auf den Acroleinreaktionsbereich aus Sicht der Edukteinspeisung und im Sinne des Stroms der Reaktanten und Reaktionsprodukte stromabwärts folgenden Druckregler handelt es sich vorzugsweise um mindestens ein, gegebenenfalls zwei oder mehrere, vorzugsweise als Druckregelventil - beispielsweise als Überströmventil - ausgebildete Druckregler. Darauf wiederum folgt stromabwärts eine Abreicherungseinheit. In einer bevorzugten Ausgestaltung der Vorrichtung kann sich der Abreicherungsbereich unmittelbar an den Druckregler anschließen. Dieses ist insbesondere bevorzugt, wenn das Abreichern des Acroleins aus der vor dem Druckregler vorliegenden Acroleinreaktionsphase durch Entspannen der Acroleinreaktionsphase erfolgt. Durch diese Maßnahmen wird ein Weiterreagieren der Acroleinphase vermindert oder gar unterbunden und damit auch die Bildung unerwünschter Nebenkomponenten.

Gemäß einer anderen Ausgestaltung der Vorrichtung kann die Abreicherungseinheit einen Wärmetauscher aufweisen. Dieser ist vorzugsweise am Beginn der Abreicherungseinheit vorgesehen. In einer anderen Ausgestaltung der Vorrichtung folgt auf den Wärmetauscher eine Abtrennvorrichtung, die als Membran oder Kristaller und insbesondere als Destillationskolonne ausgebildet ist. Es ist weiterhin vorteilhaft, dass in der Vorrichtung entweder in dem Acroleinreaktionsbereich oder vor dem Acroleinreaktionsbereich oder an beiden Stellen ein Heizelement vorgesehen ist. Dieses Heizelement wird vorzugsweise mit dem in der Abreicherungseinheit vorgesehenen Wärmetauscher thermisch gekoppelt.

Eine weitere Offenbarung betriff eine Vorrichtung zur Herstellung von wasserabsorbierenden Polymergebilden mit den Merkmalen der Vorrichtung zur Herstellung von Acrylsäure, die zusätzlich einen Polymerisationsreaktor enthält, wobei die Vorrichtung zur Herstellung von Acrylsäure fluidleitend mit dem Polymerisationsreaktor, vorzugsweise unter Zwischenschaltung der Aufarbeitungseinheit, verbunden ist.

Eine weitere Offenbarung betrifft die Herstellung wasserabsorbierender Polymergebilde durch Polymerisation der Acrylsäure, in einer bevorzugten Ausführungsform ein Verfahren zur Herstellung von Polymeren, insbesondere wasserabsorbierenden Polymergebilden durch radikalische Polymerisation von Acrylsäure, mindestens aufweisend die Schritte:
i) Bereitstellen einer ggf. teilneutralisierten Acrylsäure sowie einen Vernetzer beinhaltenden Monomerphase, wobei die Acrylsäure nach dem vorstehend beschriebenen Verfahren erhalten wird;
ii) radikalische Polymerisation der Monomerphase unter Erhalt eines Hydrogels;
iii) ggf. Zerkleinern des Hydrogels;
iv) Trocknen des Hydrogels unter Erhalt eines partikulären wasserabsorbierenden Polymergebildes;
v) ggf. Zermahlen des partikulären wasserabsorbierenden Polymergebildes;
vi) Oberflächenmodifizierung des partikulären wasserabsorbierenden Polymergebildes.

Vorzugsweise erfolgt diese radikalische Polymerisation in Gegenwart von Vernetzern sowie unter Verwendung der Acrylsäure in zumindest teilneutralisierter Form, so dass auf diese Weise vernetzte, wasserabsorbierende Polymergebilde erhalten werden. Hinsichtlich der Einzelheiten zur Herstellung solcher auf Acrylsäure basierender wasserabsorbierender Polymergebilde wird auf "Modern Superabsorbent Polymer Technology", F. L. Buchholz and A. T. Graham, Wiley-VCH-Verlag, verwiesen. Die Offenbarung dieses Lehrbuchs hinsichtlich der Einzelheiten zur Herstellung von auf vernetzten Polyacrylaten basierenden Superabsorbern wird hiermit als Referenz eingeführt und bildet einen Teil der Offenbarung der vorliegenden Erfindung. Weiterhin ist es bevorzugt, dass die Acrylsäure im Verfahrensschritt i) zu mindestens 20 mol%, besonders bevorzugt zu mindestens 50 mol%, bezogen auf das Monomer, als Salz vorliegt.

Einen Beitrag zur Lösung der eingangs genannten Aufgaben leisten auch die durch das vorstehend beschriebene Verfahren erhältlichen wasserabsorbierenden Polymergebilde.

Einen Beitrag zur Lösung der eingangs genannten Aufgaben leisten auch wasserabsorbierende Polymergebilde, welche zu mindestens 25 Gew.-%, vorzugsweise zu mindestens 50 Gew.-%, noch mehr bevorzugt zu mindestens 75 Gew.-% und am meisten bevorzugt zu mindestens 95 Gew.-%, auf Acrylsäure basieren, wobei mindestens 80 Gew.-%, vorzugsweise mindestens 90 Gew.-% und am meisten bevorzugt mindestens 95 Gew.-% der zur Herstellung der wasserabsorbierenden Polymergebilde eingesetzten Acrylsäuremonomere durch das vorstehend beschriebene Verfahren aus Glycerin über Acrolein als Zwischenprodukt erhalten wurde und die mit 0,01 bis 10 Gew.-%, bezogen auf das Gewicht der wasserabsorbierenden Polymergebilde, eines Beschichtungsmittels beschichtet worden sind, wobei als Beschichtungsmittel diejenigen Beschichtungsmittel bevorzugt sind, die bereits vorstehend im Zusammenhang mit dem Verfahren zur Herstellung wasserabsorbierender Polymergebilde genannt wurden. Vorzugsweise ist das Beschichtungsmittel kein Oberflächennachvernetzer.

Gemäß einer besonderen Ausführungsform der wasserabsorbierende Polymergebilde basieren diese zu mindestens 25 Gew.-%, vorzugsweise zu mindestens 35 Gew.-% und am meisten bevorzugt zu mindestens 45 Gew.-% auf natürlichen, biologisch abbaubaren Polymeren, vorzugsweise auf Kohlehydraten wie etwa Zellulose oder Stärke.

Einen Beitrag zur Lösung der eingangs genannten Aufgaben leisten auch Hygieneartikel, beinhaltend das vorstehend beschriebene, wasserabsorbierende Polymergebilde oder ein durch das Verfahren erhältliche, wasserabsorbierende Polymergebilde.

Einen Beitrag zur Lösung der eingangs genannten Aufgaben leistet auch die Verwendung von Acrolein, erhältlich durch das erfindungsgemäße Verfahren zur Herstellung von Acrolein, oder von Acrylsäure, erhältlich durch das erfindungsgemäße Verfahren zur Herstellung von Acrylsäure, oder von beiden Monomeren als Basis für oder in Fasern, Folien, Formassen, Textil- und Lederhilfsmittel, Flockungsmitteln, Beschichtungen, Lacken, Schäumen, Filmen, Kabeln, Dichtungsmaterialien, flüssigkeitsaufnehmenden Hygieneartikeln, insbesondere Windeln und Damenbinden, Trägern für pflanzen- oder pilzwachstumsregulierende Mittel oder Pflanzenschutzwirkstoffe, Zusätzen für Baustoffe, Verpackungsmaterialien oder Bodenzusätzen.

Einen Beitrag zur Lösung der eingangs genannten Aufgaben leisten ferner Fasern, Folien, Formassen, Textil- und Lederhilfsmittel, Flockungsmittel, Beschichtungen, Lacke, Schäume, Filme, Kabel, Dichtungsmaterialien, flüssigkeitsaufnehmenden Hygieneartikeln, insbesondere Windeln und Damenbinden, Träger für pflanzen- oder pilzwachstumsregulierende Mittel oder Pflanzenschutzwirkstoffe, Zusätze für Baustoffe, Verpackungsmaterialien oder Bodenzusätze beinhaltend oder basierend auf Acrolein, erhältlich dem erfindungsgemäßen Verfahren zur Herstellung von Acrolein oder auf Acrylsäure, erhältlich durch das erfindungsgemäße Verfahren zur Herstellung von Acrylsäure.

Einen Beitrag zur Lösung der eingangs genannten Aufgaben leistet weiterhin ein Verfahren zur Aufreinigung einer Glycerinphase G4 beinhaltend Glycerin, Wasser und von Glycerin und Wasser verschiedene Reststoffe, wobei die Glycerinphase G4 einen Glyceringehalt in einem Bereich von 20 bis 80 Gew.-%, vorzugsweise jedoch von mindestens 30 Gew.-%, noch mehr bevorzugt mindestens 40 Gew.-% und darüber hinaus bevorzugt von mindestens 50 Gew.-%, jeweils bezogen auf das Gesamtgewicht der Glycerinphase G4, aufweist und wobei die Glycerinphase G4 durch eine Nanofiltration unter Erhalt einer aufgereinigten Glycerinphase G5 unterzogen wird.

Bei der Glycerinphase G4 handelt es sich vorzugsweise um eine bereits im Zusammenhang mit dem erfindungsgemäßen Verfahren zur Herstellung von Acrolein beschriebene Rohglycerinphase. Durch die Nanofiltration wird daher dieser Rohglycerinphase mindestens teilweise mindestens ein Begleitsalz entzogen.

In diesem Zusammenhang ist es weiterhin bevorzugt, dass die Nanofiltration mittels einer polymeren Nanofiltrationsmembran erfolgt, welche vorzugsweise in einem Wickelmodul enthalten ist. Als polymere Nanofiltrationsmembranen und als Wickelmodule sind wiederum diejenigen polymeren Nanofiltrationsmembranen und Wickelmodule bevorzugt, die bereits eingangs im Zusammenhang mit dem erfindungsgemäßen Verfahren zur Herstellung von Acrolein genannt wurden.

Weiterhin ist es im Zusammenhang mit dem erfindungsgemäßen Verfahren zur Aufreinigung einer Glycerinphase G4 bevorzugt, dass das Aufreinigen bei denjenigen Druck- und Temperaturbedingungen erfolgt, die bereits im Zusammenhang mit dem erfindungsgemäßen Verfahren zur Herstellung von Acrolein, dort im Zusammenhang mit der Aufreinigung der Glycerinphase G2 oder der Mischphase M1 mittels Nanofiltration, als bevorzugte Druck- und Temperaturbedingungen genannt wurden.

Auch im Zusammenhang mit dem Verfahren zur Aufreinigung einer Glycerinphase G4 beträgt die Permeat-Ausbeute vorzugsweise mindestens 90 Gew.-%, besonders bevorzugt mindestens 95 Gew.-% und am meisten bevorzugt mindestens 99 Gew.-%.

Gemäß einer besonderen Variante des Verfahrens zur Aufreinigung einer Glycerinphase G4 beinhaltend Glycerin, Wasser und von Glycerin und Wasser verschiedene Reststoffe schließt sich an die Nanofiltration eine Behandlung des Permeates mit Aktivkohle an.

Einen Beitrag zur Lösung der eingangs genannten Aufgaben leistet auch die Verwendung einer aufgereinigten Glycerinphase G5, erhalten durch das erfindungsgemäße Verfahren zur Aufreinigung einer Glycerinphase G4, als Glycerinphase G3 in dem erfindungsgemäßen Verfahren zur Herstellung von Acrolein.

Die vorliegende Erfindung wird durch nicht limitierende Zeichnungen näher beschrieben. Die Figuren 1 bis 9 zeigen schematisch beispielhafte Ausführungsformen der vorliegenden Erfindung.
Fig. 1 zeigt schematisch eine beispielhafte Vorrichtung 1. Dabei findet in einem Reaktor (1) die Umsetzung von Glycerin zu Acrolein statt. Über Mittel (2) wird eine wässrige Glycerinphase dem Reaktor zugeführt. Die Acroleinreaktionsphase wird einer Destillationseinheit (3) zugeführt. Dabei passiert sie bevorzugt eine Neutralisationseinheit (20), durch die Lauge eingespeist wird. Das Acrolein wird nach der Destillation entnommen und kann in einem Oxidationsreaktor (10) zu Acrylsäure weiterverarbeitet werden. Die Acrylsäure kann in einem Polymerisationsreaktor (11) weiterverarbeitet werden, beispielsweise zu wasserabsorbierenden Polymeren. Über Abtrennungsmittel (4) wird die abgereicherte Acroleinreaktionsphase der Destillationseinheit (3) entnommen. Die Abtrennungsmittel (4) können einen Filter oder eine Filtrationseinheit enthalten. Die abgereicherte Acroleinreaktionsphase wird dem Reinigungsmittel (5) zugeführt. Nach der Reinigung wird die gereinigte Lösung über eine Rückführungsleitung (6) wieder dem Reaktor (1) zugeführt.
   In einer bevorzugten Ausführungsform der Erfindung können Mittel zur Einspeisung von Rohglycerin (22) zur Verfügung stehen. In einer weiteren bevorzugten Ausführungsform der Erfindung ist die ursprünglich dem Reaktor zugeführte wässrige Glycerinphase einem Reaktor (9) zur Erzeugung von Glycerin aus Glyceriden entnommen. In einer weiteren Ausführungsform der Erfindung wird das eingespeiste Glycerin vor der Einleitung in den Reaktor (1) durch Mittel zur Reinigung (7) gereinigt.

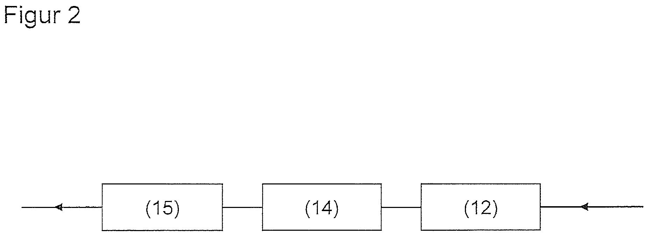

Figur 2 entspricht der Verfahrensalternative ii) des erfindungsgemäßen Verfahrens zur Herstellung von Acrolein und zeigt eine bevorzugte Ausbildung der Reinigungsmittel (5) zur Reinigung der Acroleinreaktionsphase. Dabei wird die abgereicherte Acroleinreaktionsphase über eine Filtereinheit (12), gefolgt von einer Elektrodialyseeinheit (14), gefolgt von einer Ionenaustauschereinheit (15) gereinigt. Die Filtereinheit (12) besteht bei dieser Ausführungsform bevorzugt aus einem Vorfilter in Verbindung mit einem Nanofilter oder Mitteln zur Umkehrosmose, vorzugsweise einem Nanofilter.

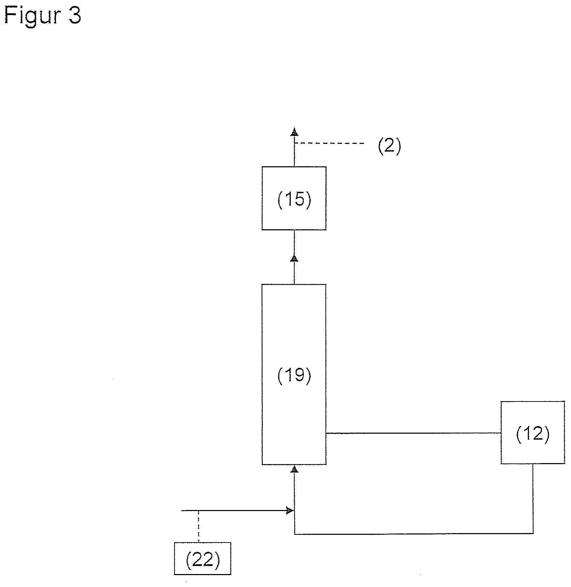

Figur 3 entspricht der Verfahrensalternative i) des erfindungsgemäßen Verfahrens zur Herstellung von Acrolein und zeigt eine bevorzugte Ausbildung der Mittel (7) zur Reinigung der Glycerinphase vor Einleitung in den Reaktor (1). Dabei wird Rohglycerin einer Destillationseinheit (19) zugeführt. Das über Destillation gereinigte Glycerin wird einer Ionenaustauschereinheit (15) zugeführt. Das so gereinigte Glycerin wird dem Reaktor (1) zugeleitet. Der Destillationsrückstand wird in einer Filtrationseinheit (12) von Schwersiedern und Salzen befreit. Die Filtrationseinheit (12) besteht aus einem Vorfilter verbunden mit einem Nanofilter und/oder einer Umkehrosmoseeinheit. Der so gereinigte Destillationsrückstand wird in einem Kreislaufprozess erneut der Destillation zugeführt.

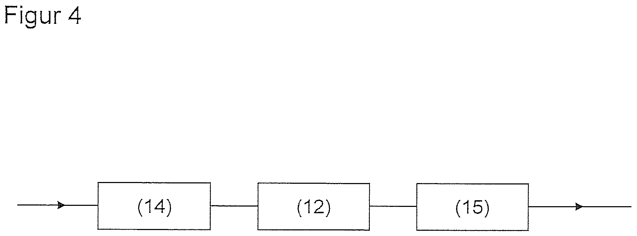

Figur 4 entspricht der Verfahrensalternative i) des erfindungsgemäßen Verfahrens zur Herstellung von Acrolein und zeigt eine weitere bevorzugte Ausbildung der Mittel (7) zur Reinigung der Glycerinphase vor Einleitung in den Reaktor (1). Das Rohglycerin wird zunächst durch eine Elektrodialyseeinheit (14) gereinigt, die einen Vorfilter enthalten kann. Anschließend erfolgt die weitere Reinigung über eine Filtrationseinheit (12), die einen Vorfilter und eine Nanofilter und/oder Mittel zur Umkehrosmose, vorzugsweise einen Nanofilter, enthält. Anschließend wird die Lösung in einer Ionenaustauschereinheit (15) gereinigt.

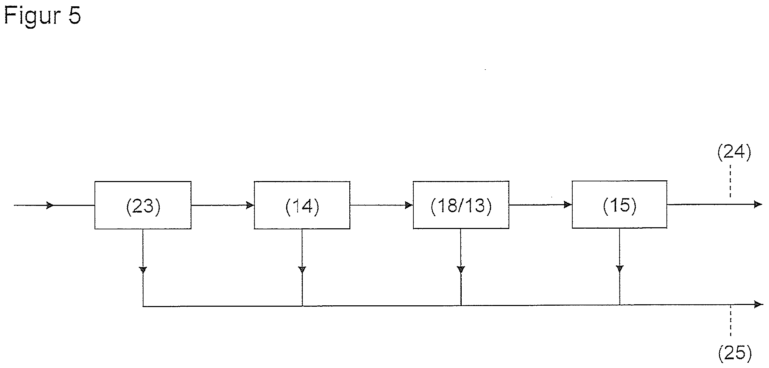

Figur 5 zeigt eine bevorzugte Ausführung erfindungsgemäß eingesetzter Reinigungsmittel (5) oder (7), die sowohl geeignet sind für die Reinigung der Acroleinreaktionsphase als auch für die Reinigung des anfänglich eingespeisten Rohglycerins (und somit geeignet ist, sowohl die Verfahrensalternative i) als auch die Verfahrensalternative ii) des erfindungsgemäßen Verfahrens zur Herstellung von Acrolein zu realisieren). Dabei wird die zu reinigende Glycerinlösung zunächst mit einem Filter (23) gefiltert, dann einer Elektrodialyseeinheit (14) zugeführt, dann mit einem Nanofilter (18) und/oder Mitteln zur Umkehrosmose (13), vorzugsweise einem Nanofilter (18), behandelt und anschließend einer Ionenaustauschereinheit (15) zugeführt. Über eine Leitung (24) wird die gereinigte Glycerinlösung entnommen. Über eine Leitung (25) werden die Nebenprodukte, wie Salze, Feststoffe und weitere Komponenten, abgeleitet.

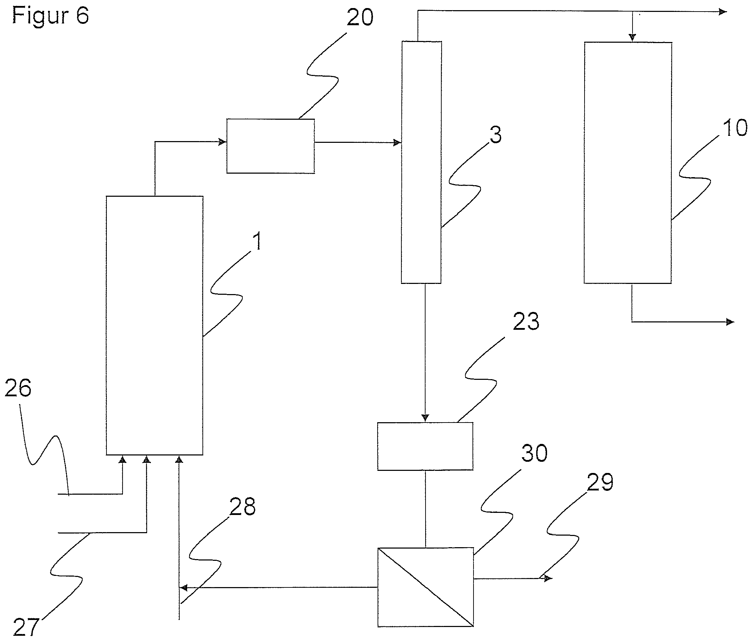

Figur 6 zeigt einen Reaktor zur Umsetzung von Glycerin zu Acrolein (1), in den eine in Inertgaseinspeisung (26), in der meist Stickstoff in den Reaktor zur Umsetzung von Glycerin (1) eingespeist wird, sowie eine Katalysatoreinspeisung (27), in der meist flüssiger Katalysator in den Reaktor zur Umsetzung von Glycerin zu Acrolein (1) eingespeist wird, münden. An den Reaktor zur Umsetzung von Glycerin zu Acrolein (1) schließt sich eine über eine Leitung mit dem Reaktor (1) verbundene Neutralisationseinheit (20) an, die wiederum über eine Leitung mit einer Destillationseinheit (3) verbunden ist. Über eine am Kopf der Destillationseinheit (3) abgehende Leitung ist diese zum Einen mit einem Oxidationsreaktor zur Oxidation von Acrylsäure (10) verbunden. Zum Anderen teilt sich diese Leitung und gibt gasförmige, aus der Destillationseinheit (3) stammende Bestandteile an die Umgebung ab. Aus dem Oxidationsreaktor zur Oxidation von Acrylsäure (10) wird die Acrylsäure bei einer weiteren Leitung zu weiteren Verwendungen, beispielsweise einem hier nicht gezeigten Polymerisationsreaktor (11), geführt. Im unteren Bereich der Destillationseinheit (3) führt eine Leitung zu einem Filter (23), an den sich ein Abscheider (30) zur Ausschleusung von Hochsiedern und Salzen anschließt, der als Nanofiltration oder auch optional als Umkehrosmose ausgestaltet sein kann und vorzugsweise als Nanofiltration ausgestaltet ist. Diese werden durch eine Hochsiederausschleusung (29) dem Kreislauf entzogen. Der Abscheider (30) ist wiederum mit einer Einspeisung für technisches Glycerin (28) verbunden, die in den Reaktor zur Umsetzung von Glycerin zu Acrolein (1) einmündet. Mittels der in Figur 6 dargestellten Vorrichtung lässt sich insbesondere die Verfahrensvariante ii) des erfindungsgemäßen Verfahrens zur Herstellung von Acrolein realisieren.

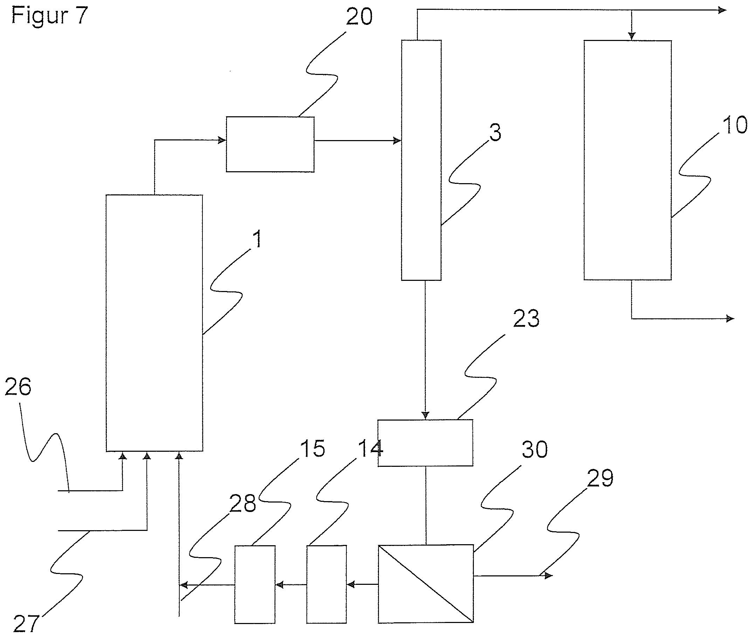

In Figur 7 sind zusätzlich zu der in Figur 6 dargestellten erfindungsgemäßen Ausgestaltung zwischen dem Abscheider (30) und der Einspeisung für technisches Glycerin nach dem Abscheider (30) eine Elektrodialyseeinheit (14), die mit einer Ionenaustauschereinheit (15) verbunden ist, die wiederum mit der Einspeise für technisches Glycerin (28) verbunden ist, vorgesehen. Auch Mittels der in Figur 7 dargestellten Vorrichtung lässt sich insbesondere die Verfahrensvariante ii) des erfindungsgemäßen Verfahrens zur Herstellung von Acrolein realisieren.

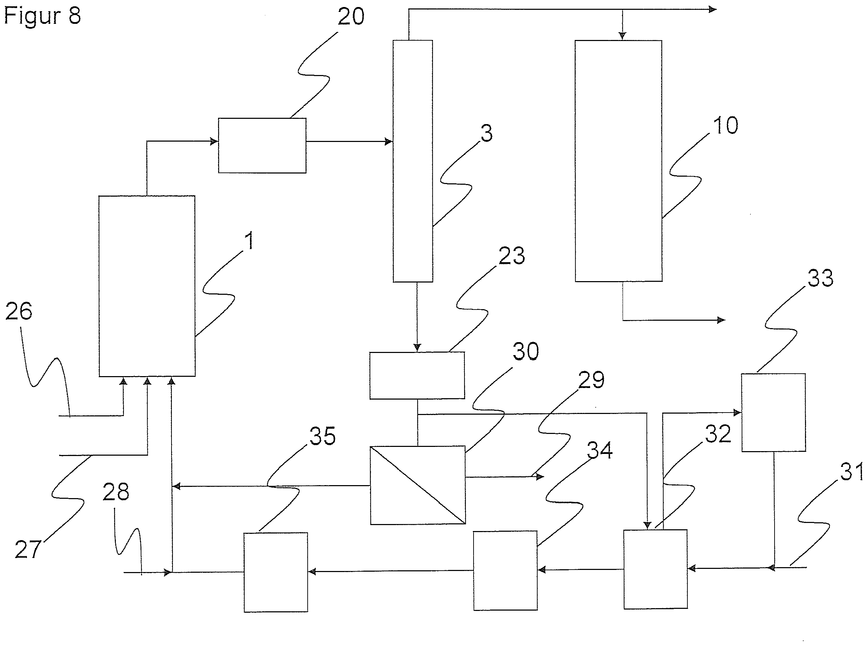

In Figur 8 wurde die in Figur 6 dargestellte erfindungsgemäße Ausgestaltung dahingehend ergänzt, dass über eine Rohglycerin-Zufuhr (31) salzhaltiges Glycerin in eine Rohglycerin-Destille (32) eingespeist wird, wobei dort ferner zumindest ein Teil des den Filter (23) verlassenden Stroms ebenso eingespeist wird. Das in der Rohglycerin-Destille (32) anfallende Sumpfprodukt wird zumindest teilweise über einen Salzabscheider (33) geführt, wobei das mindestens teilweise von Salz befreite Glycerin nach Verlassen des Salzabscheiders (33) wieder der Rohglycerin-Destille (32) zugeführt wird, auf die eine Glycerin-Destille (34) folgt. Hierbei ist es bevorzugt, dass die Bedingung, insbesondere Druck oder Temperatur oder beides, sich in der Rohglycerindestille (32) und der Glycerindestille (34) unterscheiden. Auf die Glycerindestille (34) folgt ein Rohglycerin-Ionenaustauscher (35), von dem das so gereinigte Glycerin dem Reaktor zur Umsetzung von Glycerin zu Acrolein zugeführt wird. Mittels der in Figur 8 dargestellten Vorrichtung lässt sich sowohl die Verfahrensvariante i) als auch die Verfahrensvariante ii) des erfindungsgemäßen Verfahrens zur Herstellung von Acrolein realisieren.

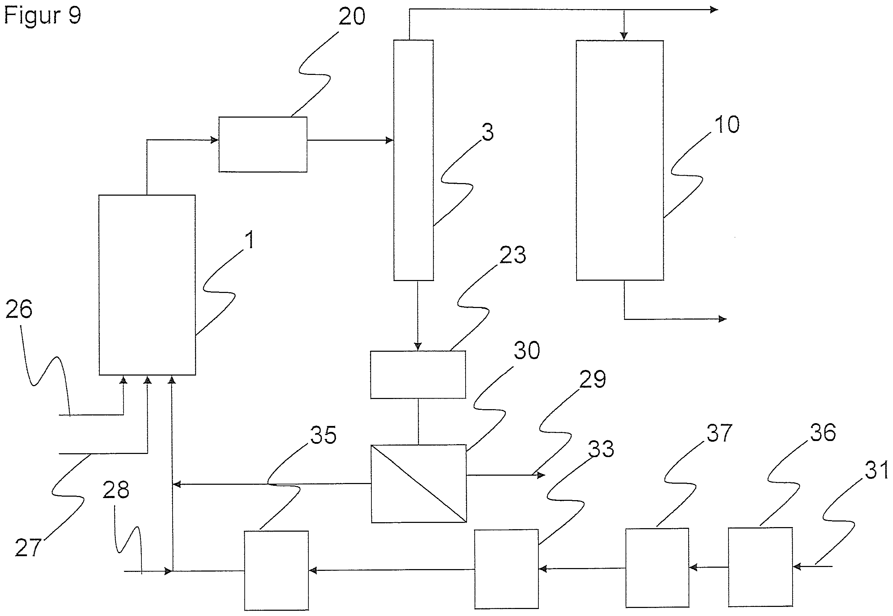

Figur 9 stellt eine Ergänzung der in Figur 6 dargestellten, erfindungsgemäßen Ausgestaltung insofern dar, als über eine Rohglycerin-Zufuhr (31) Salz beinhaltendes Rohglycerin über einen Rohglycerin-Filter (36) in eine Rohglycerin-Umkehrosmose (37) geführt wird, an den sich ein Salzabscheider (33) anschließt, der in einen Rohglycerin-Ionenaustauscher (35) mündet, der wiederum mit der Einspeise für technisches Glycerin verbunden ist. Auch Mittels der in Figur 9 dargestellten Vorrichtung lässt sich sowohl die Verfahrensvariante i) als auch die Verfahrensvariante ii) des erfindungsgemäßen Verfahrens zur Herstellung von Acrolein realisieren.

### BEZUGSZEICHENLISTE

- 1: Reaktor zur Umsetzung von Glycerin zu Acrolein
- 2: Mittel zur Zuführung einer wässrigen Glycerinphase zum Reaktor
- 3: Destillationseinheit
- 4: Abtrennungsmittel
- 5: Reinigungsmittel zur Reinigung der Acroleinreaktionsphase
- 6: Rückführungsleitung
- 7: Mittel zur Reinigung der Glycerinphase vor Einleitung in den Reaktor
- 8: Mittel zur Entnahme der abgereicherten Glycerinphase
- 9: Reaktor zur Erzeugung von Glycerin aus Glyceriden
- 10: Oxidationsreaktor zur Oxidation von Acrylsäure
- 11: Polymerisationsreaktor
- 12: Filtrationseinheit
- 13: Mittel zur Umkehrosmose
- 14: Elektrodialyseeinheit
- 15: Ionenaustauschereinheit
- 16: Mikrofilter
- 17: Ultrafilter
- 18: Nanofilter
- 19: Destillationseinheit
- 20: Neutralisationseinheit
- 21: Zuführung von Reaktionsbestandteilen
- 22: Mittel zur Einspeisung von Rohglycerin
- 23: Filter
- 24: Ableitung der Glycerinlösung
- 25: Ableitung der Salze, Feststoffe, Nebenprodukte
- 26: Inertgaseinspeisung
- 27: Katalysatoreinspeisung
- 28: Einspeisung für technisches Glycerin
- 29: Hochsiederausschleusung
- 30: Abscheider
- 31: Rohglycerin-Zufuhr
- 32: Rohglycerin-Destille
- 33: Salzabscheider
- 34: Glycerindestille
- 35: Rohglycerin-Ionentauscher
- 36: Rohglycerin-Filter
- 37: Rohglycerin-Umkehrosmose

## Patentansprüche

1. Ein Verfahren zur Herstellung von Acrolein, beinhaltend die folgenden Schritte:
(a) Dehydratisieren einer wässrigen Glycerinphase G1 in einem Acroleinreaktionsbereich unter Erhalt einer wässrigen Acroleinreaktionsphase;
(b) mindestens teilweises Auftrennen der wässrigen Acroleinreaktionsphase in eine Acrolein-reiche Acroleinphase und eine im Vergleich zu der Acroleinphase Acrolein-arme Restphase R1, wobei die Restphase R1 Glycerin, Wasser und von Glycerin und Wasser verschiedenen Reststoffe beinhaltet;
(c) Rückführen mindestens eines Teils der Restphase R1 in Schritt (a); wobei
i) aus einer Glycerinphase G2, beinhaltend Glycerin, Wasser und von Glycerin und Wasser verschiedene Reststoffe, mindestens einer dieser Reststoffe abgetrennt und die so erhaltene, aufgereinigte Glycerinphase G2 unmittelbar dem Acroleinreaktionsbereich zugeführt wird, oder
ii) aus einer Mischphase M1, erhalten durch das Vermischen einer Glycerinphase G2, beinhaltend Glycerin, Wasser und von Glycerin und Wasser verschiedene Reststoffe, mit der Acrolein-armen Restphase R1, mindestens einer der von Glycerin und Wasser verschiedenen Reststoffe abgetrennt und die so erhaltene, aufgereinigte Mischphase M1 dem Acroleinreaktionsbereich zugeführt wird,
wobei neben der aufgereinigten Glycerinphase G2 oder der aufgereinigten Mischphase M1 eine weitere Glycerinphase G3 dem Acroleinreaktionsbereich zugeführt wird, wobei die Glycerinphase G3 einen höheren Glyceringehalt als die Glycerinphase G2, und wobei die Glycerinphase G3 einen geringeren Salzgehalt als die Glycerinphase G2 und wobei die Glycerinphase G3 einen geringeren Gehalt an von Glycerin verschiedenen organischen Verbindungen als die Glycerinphase G2 aufweist, und wobei die Glycerinphase G3 als Destillat bei der Destillation von bei der Spaltung oder der Umesterung von Triglyceriden erhaltenem Rohglycerin erhalten wird und mindestens ein Teil des bei der Destillation des Rohglycerins anfallenden Sumpfproduktes einen Salzabscheider durchläuft und anschließend der Destillation des Rohglycerins wieder zugeführt wird.

2. Ein Verfahren zur Herstellung von Acrylsäure, beinhaltend die Schritte:
(A1) Bereitstellen einer Acrolein-reichen Acroleinphase nach einem Verfahren nach Anspruch 1;
(A2) Oxidation der Acrolein-reichen Acroleinphase aus Schritt (A1) unter Erhalt einer Acrylsäurephase; und
(A3) gegebenenfalls Aufarbeiten der Acrylsäurephase unter Erhalt von Acrylsäure.

3. Ein Verfahren zur Herstellung eines Polymers, beinhaltend die Schritte:
(P1) Bereitstellen einer Acrylsäurephase oder einer Acrylsäure nach einem Verfahren gemäß Anspruch 2 in einer Polymeristionsphase;
(P2) Polymerisieren der Polymerisationsphase unter Erhalt eines Polymers.

4. Verfahren nach einem der Ansprüche 1 bis 3, wobei die Glycerinphase G2 eine Rohglycerinphase ist, welche als ein Reaktionsprodukt bei der Spaltung oder der Umesterung von Triglyceriden erhalten wird.

5. Verfahren nach einem der Ansprüche 1 bis 4, wobei die Glycerinphase G3 oder die Glycerinphase G2 oder beide Glycerinphasen als Reststoffe Salze, Fettsäuren, Seifen, Monoglyceride, Diglyceride, Triglyceride und Kondensationsprodukte des Glycerins beinhalten.

6. Verfahren nach einem der vorhergehenden Ansprüche, wobei das Abtrennen gemäß den Alternativen i) oder ii) durch mindestens eine der folgenden Abtrennarten erfolgt:
α1 Destillation;
α2 Filtration, ausgewählt ist aus der Gruppe bestehend aus einer Mikrofiltration, einer Ultrafiltration, einer Umkehrosmose, einer Nanofiltration oder mindestens zwei davon vorzugsweise Nanofiltration oder Umkehrosmose oder beides erfolgt;
α3 Elektrodialyse;
α4 Ionenaustauscherbehandlung.

7. Verfahren nach einem der vorhergehenden Ansprüche, wobei beim Abtrennen gemäß den Alternativen i) oder ii) mindestens teilweise mindestens ein Begleitsalz entzogen wird.

8. Verfahren nach einem der Ansprüche 6 bis 7, wobei das Abtrennen mit einer Permeat-Ausbeute von mindestens 90 Gew.-% durchgeführt wird, wobei das Abtrennen mittels der polymeren Nanofiltrationsmembran bei einer Temperatur von mehr als 40°C und bei einem Transmembrandruck von mehr als 30 bar erfolgt.

9. Verfahren nach einem der vorhergehenden Ansprüche, wobei das Abtrennen gemäß den Alternativen i) oder ii) durch eine Filtration, gefolgt von einer Elektrodialyse erfolgt.

10. Verfahren nach Anspruch 6, wobei auf die Elektrodialyse eine osmotische Reinigung oder auf die Elektrodialyse oder die osmotische Reinigung eine lonenaustauscherbehandlung folgt.

11. Verfahren nach einem der vorhergehenden Ansprüche, wobei ein Teil des Glycerins der Glycerinphase G1 in dem Verfahren zur Herstellung von Acrolein in einem Kreislauf geführt wird.

12. Verfahren nach einem der vorhergehenden Ansprüche, wobei der Acroleinreaktionsbereich einen Dehydratisierungskatalysator beinhaltet.

13. Verfahren nach einem der vorhergehenden Ansprüche, wobei die Acroleinreaktionsphase eine von Wasser verschiedene Flüssigkeit aufweist.

14. Verfahren nach einem der vorhergehenden Ansprüche, wobei der Acroleinreaktionsbereich ein Metall oder eine Metallverbindungen oder beides beinhaltet.

15. Verfahren nach einem der vorhergehenden Ansprüche, wobei die Acroleinreaktionsphase am Ende des Acroleinreaktionsbereichs einen Anteil von weniger als 50 Gew.-%, bezogen auf die Acroleinreaktionsphase, Glycerin beinhaltet.

16. Verfahren nach einem der vorhergehenden Ansprüche, wobei die Acroleinreaktionsphase am Ende des Acroleinreaktionsbereichs einen Anteil im Bereich von 0,1 bis 50 Gew.-%, bezogen auf die Acroleinreaktionsphase, Acrolein beinhaltet.

17. Verfahren nach einem der vorhergehenden Ansprüche, wobei die Acroleinreaktionsphase vor dem Abreichern gemäß Verfahrensschritt (b) unter einem höheren Druck als bei dem Abreichern steht.

18. Verfahren nach einem der vorhergehenden Ansprüche, wobei die Acroleinkonzentration in der Acroleinreaktionsphase vor dem Abreichern gemäß Verfahrensschritt (b) um mindestens 5 % höher ist als nach dem Abreichern.

19. Verfahren nach einem der vorhergehenden Ansprüche, wobei ein Schleppgas eingesetzt wird.

20. Verfahren zur Herstellung von Acrylsäure nach einem der Ansprüche 2 bis 19, wobei eine Vorrichtung zur Herstellung von Acrolein eingesetzt wird, beinhaltend fluidleitend miteinander verbunden
- einen Reaktor (1) zur Umsetzung von Glycerin in einer Glycerinphase zu Acrolein,
- Mittel (2) zur Zuführung einer wässrigen Glycerinphase zu dem Reaktor (1),
- eine Destillationseinheit (3) zur Abreicherung von Acrolein,
- Abtrennungsmittel (4) zur Abtrennung der abgereicherten Acroleinphase,
- Reinigungsmittel (5) zur Reinigung der abgereicherten Acroleinphase oder zur Reinigung der wässrigen Glycerinphase, und
- eine Rückführungsleitung (6), die die Abtrennungsmittel (4), die Reinigungsmittel (5) und den Reaktor (1) fluidleitend miteinander verbindet.

21. Verfahren nach Anspruch 20, wobei die Reinigungsmittel (5) ausgewählt sind aus einer Filtrationseinheit (12), Mitteln zur Umkehrosmose (13), einer Elektrodialyseeinheit (14), einer Ionenaustauschereinheit (15) oder einer Kombination aus mindestens zwei davon, wobei die Filtrationseinheit mindestens einen Mikrofilter (16), einen Ultrafilter (17), einen Nanofilter (18) oder eine Kombination aus mindestens zwei davon beinhaltet, bevorzugt ist das Reinigungsmittel (5) ein Nanofilter (18), wobei der Nanofilter (18) eine polymere Nanofiltrationsmembran beinhaltet, wobei der Nanofilter aus einem die polymere Nanofiltrationsmembran beinhaltenden Wickelmodul gefertigt ist und das Wickelmodul einen Feedspacer von mindestens 40 mm aufweist und die polymere Nanofiltrationsmembran aus einem Polyamid oder einem Polyethersulfon gefertigt ist.

22. Verfahren nach einem der Ansprüche 20 bis 21, beinhaltend einen Reaktor (9) zur Erzeugung von Glycerin aus Glyceriden.

23. Verfahren zur Herstellung von Acrylsäure nach einem der Ansprüche 20 bis 22, wobei die Vorrichtung zur Herstellung von Acrolein fluidleitend mit einem Oxidationsreaktor (10) zur Oxidation von Acrolein verbunden ist.

## Claims

1. Process for producing acrolein, comprising the following steps:
(a) dehydrating an aqueous glycerol phase G1 in an acrolein reaction region to give an aqueous acrolein reaction phase;
(b) at least partially separating the aqueous acrolein reaction phase into an acrolein-rich acrolein phase and a residual phase R1 that is depleted in acrolein compared to the acrolein phase, wherein the residual phase R1 comprises glycerol, water and residues other than glycerol and water;
(c) recirculating at least part of the residual phase R1 to step (a); wherein
i) at least one of these residues is separated off from a glycerol phase G2 comprising glycerol, water and residues other than glycerol and water, and the purified glycerol phase G2 obtained in this way is fed directly to the acrolein reaction region, or
ii) at least one of the residues other than glycerol and water is separated off from a mixed phase M1, obtained by mixing a glycerol phase G2, comprising glycerol, water and residues other than glycerol and water, with the acrolein-depleted residual phase R1, and the purified mixed phase M1 obtained in this way is fed to the acrolein reaction region,
wherein, in addition to the purified glycerol phase G2 or the purified mixed phase M1, a further glycerol phase G3 is fed to the acrolein reaction region, wherein the glycerol phase G3 has a higher glycerol content than the glycerol phase G2, and wherein the glycerol phase G3 has a lower salt content than the glycerol phase G2 and wherein the glycerol phase G3 has a lower content of organic compounds other than glycerol than the glycerol phase G2, and wherein the glycerol phase G3 is obtained as a distillate in the distillation of crude glycerol obtained in the cleavage or transesterification of triglycerides and at least part of the bottom product obtained in the distillation of the crude glycerol passes through a salt separator and is subsequently fed back to the distillation of the crude glycerol.

2. Process for producing acrylic acid, comprising the steps of:
(A1) providing an acrolein-rich acrolein phase by a process according to Claim 1;
(A2) oxidizing the acrolein-rich acrolein phase from step (A1) to give an acrylic acid phase; and
(A3) optionally processing the acrylic acid phase to give acrylic acid.

3. Process for producing a polymer, comprising the steps of:
(P1) providing an acrylic acid phase or an acrylic acid by a process according to Claim 2 in a polymerization phase;
(P2) polymerizing the polymerization phase to give a polymer.

4. Process according to any of Claims 1 to 3, wherein the glycerol phase G2 is a crude glycerol phase which is obtained as a reaction product in the cleavage or transesterification of triglycerides.

5. Process according to any of Claims 1 to 4, wherein the glycerol phase G3 or the glycerol phase G2 or both glycerol phases comprise salts, fatty acids, soaps, monoglycerides, diglycerides, triglycerides and glycerol condensation products as residues.

6. Process according to any of the preceding claims, wherein the separation according to alternative i) or ii) is effected by means of at least one of the following separation methods:
α1 distillation;
α2 filtration, which is selected from the group consisting of microfiltration, ultrafiltration, reverse osmosis, nanofiltration or at least two thereof, preferably nanofiltration or reverse osmosis or both;
α3 electrodialysis;
α4 ion exchange treatment.

7. Process according to any of the preceding claims, wherein at least one accompanying salt is at least partially removed in the separation according to alternative i) or ii).

8. Process according to either of Claims 6 and 7, wherein the separation is carried out with a permeate yield of at least 90% by weight, wherein the separation by means of the polymeric nanofiltration membrane is conducted at a temperature of more than 40°C and a transmembrane pressure of more than 30 bar.

9. Process according to any of the preceding claims, wherein the separation according to alternative i) or ii) is carried out by means of filtration, followed by electrodialysis.

10. Process according to Claim 6, wherein the electrodialysis is followed by osmotic purification or the electrodialysis or osmotic purification is followed by ion exchange treatment.

11. Process according to any of the preceding claims, wherein part of the glycerol of the glycerol phase G1 is circulated in the process for producing acrolein.

12. Process according to any of the preceding claims, wherein the acrolein reaction region comprises a dehydration catalyst.

13. Process according to any of the preceding claims, wherein the acrolein reaction phase comprises a liquid other than water.

14. Process according to any of the preceding claims, wherein the acrolein reaction region comprises a metal or a metal compound or both.

15. Process according to any of the preceding claims, wherein the acrolein reaction phase at the end of the acrolein reaction region comprises a proportion of glycerol of less than 50% by weight, based on the acrolein reaction phase.

16. Process according to any of the preceding claims, wherein the acrolein reaction phase at the end of the acrolein reaction region comprises a proportion of acrolein in the range of 0.1 to 50% by weight, based on the acrolein reaction phase.

17. Process according to any of the preceding claims, wherein the acrolein reaction phase prior to the depletion according to process step (b) is under a higher pressure than during the depletion.

18. Process according to any of the preceding claims, wherein the acrolein concentration in the acrolein reaction phase prior to the depletion according to process step (b) is at least 5% higher than after the depletion.

19. Process according to any of the preceding claims, wherein an entrainment gas is used.

20. Process for producing acrylic acid according to any of Claims 2 to 19, wherein an apparatus for producing acrolein is used, which comprises, connected in a fluid-conducting manner,
- a reactor (1) for converting glycerol in a glycerol phase into acrolein,
- means (2) of feeding an aqueous glycerol phase to the reactor (1),
- a distillation unit (3) for depleting acrolein,
- separation means (4) for separating off the depleted acrolein phase,
- purification means (5) for purifying the depleted acrolein phase or for purifying the aqueous glycerol phase, and
- a return line (6) which connects the separation means (4), the purification means (5) and the reactor (1) in a fluid-conducting manner.

21. Process according to Claim 20, wherein the purification means (5) are selected from among a filtration unit (12), means for reverse osmosis (13), an electrodialysis unit (14), an ion exchange unit (15) or a combination of at least two thereof, wherein the filtration unit comprises at least one microfilter (16), an ultrafilter (17), a nanofilter (18) or a combination of at least two thereof, the purification means (5) preferably being a nanofilter (18), wherein the nanofilter (18) comprises a polymeric nanofiltration membrane, wherein the nanofilter is made of a wound module comprising the polymeric nanofiltration membrane and the wound module has a feed spacer of at least 40 mm and the polymeric nanofiltration membrane is made of a polyamide or a polyether sulfone.

22. Process according to either of Claims 20 and 21, comprising a reactor (9) for producing glycerol from glycerides.

23. Process for producing acrylic acid according to any of Claims 20 to 22, wherein the apparatus for producing acrolein is connected in a fluid-conducting manner to an oxidation reactor (10) for oxidizing acrolein.

## Revendications

1. Procédé de fabrication d'acroléine, contenant les étapes suivantes :
(a) la déshydratation d'une phase glycérine aqueuse G1 dans une zone de réaction de l'acroléine pour obtenir une phase réactionnelle acroléine aqueuse ;
(b) la séparation au moins partielle de la phase réactionnelle acroléine aqueuse en une phase acroléine riche en acroléine et une phase résiduelle R1 pauvre en acroléine en comparaison de la phase acroléine, la phase résiduelle R1 contenant de la glycérine, de l'eau et des substances résiduelles différentes de la glycérine et de l'eau ;
(c) le recyclage d'au moins une partie de la phase résiduelle R1 dans l'étape (a) ;
i) à partir d'une phase glycérine G2, contenant de la glycérine, de l'eau et des substances résiduelles différentes de la glycérine et de l'eau, au moins une de ces substances résiduelles étant séparée et la phase glycérine G2 purifiée ainsi obtenue étant introduite directement dans la zone de réaction de l'acroléine, ou
ii) à partir d'une phase mixte M1, obtenue par le mélange d'une phase glycérine G2, contenant de la glycérine, de l'eau et des substances résiduelles différentes de la glycérine et de l'eau, avec la phase résiduelle R1 pauvre en acroléine, au moins une des substances résiduelles différentes de la glycérine et de l'eau étant séparée et la phase mixte M1 purifiée ainsi obtenue étant introduite dans la zone de réaction de l'acroléine,
en plus de la phase glycérine purifiée G2 ou de la phase mixte purifiée M1, une phase glycérine supplémentaire G3 étant introduite dans la zone de réaction de l'acroléine, la phase glycérine G3 présentant une teneur en glycérine plus élevée que la phase glycérine G2 et la phase glycérine G3 présentant une teneur en sels plus faible que la phase glycérine G2 et la phase glycérine G3 présentant une teneur en composés organiques différents de la glycérine plus faible que la phase glycérine G2, et la phase glycérine G3 étant obtenue en tant que distillat lors de la distillation de glycérine brute obtenue lors du clivage ou de la transestérification de triglycérides, et au moins une partie du produit de fond formé lors de la distillation de la glycérine brute traversant un séparateur de sels, puis étant réintroduite dans la distillation de la glycérine brute.

2. Procédé de fabrication d'acide acrylique, contenant les étapes suivantes :
(A1) la préparation d'une phase acroléine riche en acroléine par un procédé selon la revendication 1 ;
(A2) l'oxydation de la phase acroléine riche en acroléine de l'étape (A1) pour obtenir une phase acide acrylique ; et
(A3) éventuellement le traitement de la phase acide acrylique pour obtenir de l'acide acrylique.

3. Procédé de fabrication d'un polymère, contenant les étapes suivantes :
(P1) la préparation d'une phase acide acrylique ou d'un acide acrylique par un procédé selon la revendication 2 dans une phase de polymérisation ;
(P2) la polymérisation de la phase de polymérisation pour obtenir un polymère.

4. Procédé selon l'une quelconque des revendications 1 à 3, dans lequel la phase glycérine G2 est une phase glycérine brute, qui est obtenue en tant que produit de réaction lors du clivage ou de la transestérification de triglycérides.

5. Procédé selon l'une quelconque des revendications 1 à 4, dans lequel la phase glycérine G3 ou la phase glycérine G2 ou les deux phases glycérine contiennent en tant que substances résiduelles des sels, des acides gras, des savons, des monoglycérides, des diglycérides, des triglycérides et des produits de condensation de glycérine.

6. Procédé selon l'une quelconque des revendications précédentes, dans lequel la séparation selon les variantes i) ou ii) a lieu par au moins un des types de séparation suivants :
α1 une distillation ;
α2 une filtration, choisie dans le groupe constitué par une microfiltration, une ultrafiltration, une osmose inverse, une nanofiltration ou au moins deux d'entre elles, de préférence une nanofiltration ou une osmose inverse ou les deux ;
α3 une électrodialyse ;
α4 un traitement par échange d'ions.

7. Procédé selon l'une quelconque des revendications précédentes, dans lequel au moins un sel associé est au moins partiellement extrait lors de la séparation selon les variantes i) ou ii).

8. Procédé selon l'une quelconque des revendications 6 à 7, dans lequel la séparation est réalisée avec un rendement en perméat d'au moins 90 % en poids, la séparation ayant lieu au moyen de la membrane de nanofiltration polymère à une température de plus de 40 °C et à une pression transmembranaire de plus de 30 bar.

9. Procédé selon l'une quelconque des revendications précédentes, dans lequel la séparation selon les variantes i) ou ii) a lieu par une filtration, suivie par une électrodialyse.

10. Procédé selon la revendication 6, dans lequel l'électrodialyse est suivie par une purification osmotique ou l'électrodialyse ou la purification osmotique est suivie par un traitement par échange d'ions.

11. Procédé selon l'une quelconque des revendications précédentes, dans lequel une partie de la glycérine de la phase glycérine G1 est mise en circulation dans un circuit dans le procédé de fabrication d'acroléine.

12. Procédé selon l'une quelconque des revendications précédentes, dans lequel la zone de réaction de l'acroléine contient un catalyseur de déshydratation.

13. Procédé selon l'une quelconque des revendications précédentes, dans lequel la phase réactionnelle acroléine comprend un liquide différent de l'eau.

14. Procédé selon l'une quelconque des revendications précédentes, dans lequel la zone de réaction de l'acroléine contient un métal ou un composé métallique ou les deux.

15. Procédé selon l'une quelconque des revendications précédentes, dans lequel la phase réactionnelle acroléine contient à la fin de la zone de réaction de l'acroléine une proportion inférieure à 50 % en poids, par rapport à la phase réactionnelle acroléine, de glycérine.

16. Procédé selon l'une quelconque des revendications précédentes, dans lequel la phase réactionnelle acroléine contient à la fin de la zone de réaction de l'acroléine une proportion dans la plage allant de 0,1 à 50 % en poids, par rapport à la phase réactionnelle acroléine, d'acroléine.

17. Procédé selon l'une quelconque des revendications précédentes, dans lequel la phase réactionnelle acroléine se trouve avant l'appauvrissement selon l'étape de procédé (b) sous une pression plus élevée que lors de l'appauvrissement.

18. Procédé selon l'une quelconque des revendications précédentes, dans lequel la concentration en acroléine dans la phase réactionnelle acroléine avant l'appauvrissement selon l'étape de procédé (b) est au moins 5 % supérieure à celle après l'appauvrissement.

19. Procédé selon l'une quelconque des revendications précédentes, dans lequel un gaz d'entraînement est utilisé.

20. Procédé de fabrication d'acide acrylique selon l'une quelconque des revendications 2 à 19, dans lequel un dispositif pour la fabrication d'acroléine est utilisé, contenant, reliés les uns aux autres par connexion fluidique :
- un réacteur (1) pour la mise en réaction de glycérine dans une phase glycérine pour former de l'acroléine,
- des moyens (2) pour l'introduction d'une phase glycérine aqueuse dans le réacteur (1),
- une unité de distillation (3) pour l'appauvrissement de l'acroléine,
- des moyens de séparation (4) pour la séparation de la phase acroléine appauvrie,
- des moyens de purification (5) pour la purification de la phase acroléine appauvrie ou pour la purification de la phase glycérine aqueuse, et
- une conduite de recyclage (6) qui raccorde fluidiquement les uns aux autres les moyens de séparation (4), les moyens de purification (5) et le réacteur (1).

21. Procédé selon la revendication 20, dans lequel les moyens de purification (5) sont choisis parmi une unité de filtration (12), des moyens d'osmose inverse (13), une unité d'électrodialyse (14), une unité d'échange d'ions (15) ou une combinaison d'au moins deux d'entre eux, l'unité de filtration contenant au moins un microfiltre (16), un ultrafiltre (17), un nanofiltre (18) ou une combinaison d'au moins deux d'entre eux, le moyen de purification (5) étant de préférence un nanofiltre (18), le nanofiltre (18) contenant une membrane de nanofiltration polymère, le nanofiltre étant fabriqué à partir d'un module enroulé contenant la membrane de nanofiltration polymère et le module enroulé présentant un espaceur d'alimentation d'au moins 40 mm et la membrane de nanofiltration polymère étant fabriquée en un polyamide ou une polyéther-sulfone.

22. Procédé selon l'une quelconque des revendications 20 à 21, contenant un réacteur (9) pour la formation de glycérine à partir de glycérides.

23. Procédé de fabrication d'acide acrylique selon l'une quelconque des revendications 20 à 22, dans lequel le dispositif pour la fabrication d'acroléine est relié par connexion fluidique avec un réacteur d'oxydation (10) pour l'oxydation d'acroléine.
